# EUROPEAN PATENT APPLICATION

(11) **EP 2 330 123 A2**
(43) Date of publication of application: **08.06.2011**
(21) Application number: 10185349.7
(22) Date of filing: 28.12.2005
(51) Int. Cl.: C07K 14/47, C07K 14/52, C07K 14/475, C12N 9/78, C12N 9/12, C12N 15/12, C12Q 1/68, C12Q 1/70, C07K 16/18, A61K 38/00, A61K 48/00

(54) **HIV resistance genes**

(30) Priority: 24.12.2004 GB 0428301
(62) Divisional of application: 05823021.0
(71) Applicant: ImmunoClin Limited, London, Greater London N12 8NP (GB); Osaka Industrial Promotion Organization, Osaka-shi, Osaka 540-0029 (JP); Toppan Printing Company Limited, Tokyo 101-0024 (JP)
(72) Inventor: Clerici, Mario, N12 8NP, London (IT); MIYAZAWA, Masaaki, Osaka-Sayama Osaka 589-8511 (JP); IRIE, Shinji, Chiyoda-ku Tokyo 101-0024 (JP)
(74) Representative: Heaton, Joanne Marie

(57) **Abstract**

Genes involved in immune resistance to infection and uses thereof are described. In particular genes which are involved in resistance to HIV infection and in slowing disease progression in infected individuals are described.

## Description

This invention relates to resistance genes, and to uses thereof. More particularly, the present invention relates to genes involved in immune resistance to infection.

It is known in the art that it is possible to diagnose a predisposition to certain diseases with the use of marker genes. For example, oncogenes or tumour suppressor genes are widely regarded as being indicative of a susceptibility to certain cancers, especially in view of the associations between mutated oncogenes and deleted tumour suppressor genes and certain cancers. Additionally, genes have been identified, such as the BRCA genes, which are taken to be predictive of a greater risk of contracting cancers, for example breast cancers. It is also known that some individuals are highly susceptible or resistant to infection, especially viral infection. Prediction of disease susceptibility is beneficial for those possessing predisposing genes in order to avoid unnecessary contacts with known aetiological agents, chemicals, or viruses, and to take known and developing preventative means. It is also useful in the design of a vaccine against viral disease or for gene therapy. In addition, prediction of the speed of disease progression may allow opportunity for individualized, more efficient management of therapy.

Additionally, the development of an effective vaccine against major viral diseases such as human immunodeficiency virus (HIV) infection is a pressing matter with global socio-economic ramifications. HIV is the causative agent of acquired immunodeficiency syndrome (AIDS). One of the keys to the development of such a vaccine is the understanding of the mechanisms of natural resistance against HIV infection. In this regard, the absence of clinical progression in some HIV-1-infected individuals and the lack of detectable HIV-1 genome despite multiple and repeated exposure to this virus in some apparently resistant groups of people are two notable phenomena when considering the development of preventative and therapeutic means to HIV infection. Several host genes have been associated with possible resistance against HIV infection and with either delayed or accelerated development of AIDS after HIV seroconversion [reviewed in 1]. These host genes include genes encoding chemokine receptors and cytokines, killer immunoglobulin-like receptors (KIRs) that serve as natural killer cell receptors, and those within the major histocompatibility complex (MHC) [1 -11].

Some of the individuals who are naturally resistant possess a mutated HIV co-receptor gene known as CCR5Δ32 [1-5] However, this mutation is recessive and the homozygosity that confers resistance against HIV entry into cells is only rarely found. Thus, the above mutation cannot account for the majority of individuals who show spontaneous resistance against HIV infection. Among existing human clusters showing natural resistance against HIV infection, there is a distinct group of people known as HIV-exposed seronegatives (ESNs) or as HIV-1-exposed but uninfected individuals (EUls) who have evidence of multiple and repeated exposure to HIV, but nevertheless possess no serum IgG antibodies reactive to HIV [12, 13]. EUls show strong HIV-1 antigen-specific T-lymphocyte responses and HIV-1-reactive mucosal IgA production despite the absence of detectable plasma HIV-1 RNA and HIV-1 cDNA from peripheral blood mononuclear cells (PBMCs) [14-16]. Detection of HIV antigen-specific T-lymphocyte responses and of HIV-reactive IgA antibodies in urethral or vaginal secretions from these ESNs/EUIs indicate that they have been exposed to HIV but the exposure has not resulted in infection [12-17]. Attempts to associate the ESN/EUI status with the previously reported genetic polymorphisms have so far been unsuccessful [10, 14]. Demonstration of HIV-1-neutralizing activity exerted by the mucosal IgA isolated from EUls [17-19] has suggested that rapid production and class switching of HIV-1-neutralizing antibodies might contribute to the presumable immune resistance against HIV infection. Protective roles of neutralizing antibodies against HIV-1-related simian immunodeficiency virus (SIV) or pathogenic chimeras between HIV-1 and SIV have also been demonstrated by passive transfer and vaccine-induced active immunization experiments in non-human primates [20-23]. However, the degree of protection afforded by the generation of various HIV-specific immune responses in humans has not been established and neither immunological nor genetic correlates of presumable protection against HIV infection are currently known.

It was recently shown that APOBEC3G, a cellular enzyme belonging to the apolipoprotein B mRNA-editing enzyme catalytic polypeptide-like (APOBEC) family cytidine deaminases, has a broad antiretroviral activity [24-27]. Thus, after the penetration of HIV into target cells and the initiation of reverse transcription of viral genomic RNA into DNA, APOBEC3G induces the conversion of cytosine to uracil in minus strand cDNA leading to a failure of reverse transcriptase and to a very high number of G-to-A mutation in the integrated proviral genome that greatly reduces viral fitness [24, 25, 28]. HIV Vif protein counteracts the activity of APOBEC3G by forming a complex with it in the cytoplasm and by impeding its packaging into virions, thus preventing editing mutations upon entry of the newly generated viral particles into target cells [29, 30]. The interaction with Vif stimulates APOBEC3G degradation by ubiquitine-proteasome pathway [30-33] and increases viral replication. This explains the biological properties of Vif which are to facilitate HIV replication and enhance the infectivity of progeny virions 10- to 100-fold. The importance of the Vif-APOBEC interplay in determining HIV infectiousness is further strengthened by the observation that cell lines that are permissive to the replication of *vif*-deleted HIV do not express APOBEC3G [29, 34].

Even more recently, a second DNA-editing enzyme, APOBEC3F, was found to be involved in the resistance of human cells against HIV infection [35-37]. APOBEC3F is also packaged into HIV virions and inhibits their infectivity by specifically binding to the Vif protein. APOBEC3G and APOBEC3F are co-expressed in non-permissive human cells where they form heterodimers [37]. Importantly, the antiviral activity of APOBEC3F is partially resistant to Vif, resulting in a more pronounced 5'GA-to-5'AA bias, and thus in a stronger impairment of HIV replication [38]. However, there is no known direct effect of APOBEC3G and APOBEC3F on immune cells, and the possible differences in the expression of these DNA mutator proteins have not been associated with the stronger and/or earlier immune responses upon HIV exposure observed in the above ESNs/EUls.

In a mouse model, resistance to Friend murine leukaemia virus (FV) is controlled by a number of genetic factors, and complex immune responses, including B, T and NK cell responses, are required for efficient protection and survival of the animals. See Table 1 below.

**Table 1. Identified host gene loci that influence FV replication in target cells and immune response to FV antigens**

| **Gene locus** | **Chromo-somal location** | **Resistant allele(s)** | **Susceptible allele(s)** | **Phenotype(s) influenced** | **Functional homologue in HIV infection** |
|---|---|---|---|---|---|
| *mCAT-1* | 5 | *null* | +/+, +/- | Viral attachment and entry into target cells | *CCD5Δ32* (homo) |
| *Fv4* | 12 | *r*/*r, r*/*s* (some wild mice) | *s*/*s* (most laboratory strains) | Block cell-surface receptor (mCAT-1) | |
| *Fv2* | 9 | *r*/*r* (C57BL) | *r*/*s, sls* (most other strains) | Uncouple growth signalling through the STK receptor | |
| *Fv1* | 4 | *b* to N-tropic viruses *n* to B-tropic viruses | *b*/*b* to B-tropic viruses *n*/*n* to N-tropic viruses | | TRIM5α |
| *Rfv3* | 15 | *r*/*r, r*/*s* (C57BL and its F₁ progenies) | *s*/*s* (A/WySn) | Recovery from viremia, kinetics of neutralizing antibody production | 22q13.1 |
| *Rfv1* | 17 (*H2D*) | *D^{b}* | *D^{d}, D^{k}, D^{q}, D^{dm14}* | Cytokine production from T cells | HLA B*35-Cw*04 HLA class I homozygosity |
| *Rfv2* | 17 (*Q*/*TL*) | *Qa1*^{a} | *Qal^{b}* | NK susceptibility of infected cells (?) | KIR3DS1 MICA, MICB |
| *H2A* | 17 | *A^{b}* | *A^{d}, A^{k}, A^{bm12}* | CD4⁺ T cell responses to viral antigens | |
| *H2E* | 17 | *E^{b}* | *E^{d}, E^{k}* | CD4⁺ T cell responses to viral antigens | |

By studying the *Rfv3* locus in mice and DNA samples from EUI individuals, with their informed consent, the inventors found that EUls possess distinct rare alleles at microsatellite loci within a region of human chromosome 22 that is syntenic to the area of mouse chromosome 15 containing the retrovirus resistance gene, *Rfv3.* In International Patent Application No WO 2004/035825 the inventors described specific genotypes or polymorphisms which are associated with resistance to HIV infection in the ESNs/EUIs in European individuals (in Italy).

The present inventors have now identified the genes and their regulatory elements implicated in naturally acquired immune resistance against the establishment of HIV infection known as the ESN or EUI status. The results described herein suggest that genes and their regulatory elements now identified by the inventors represent the genetic factor(s) that allow some people to mount anti-HIV immune responses upon exposure to HIV. It is likely that the combination of some of these factors is linked to the fact that some individuals take much longer to progress to AIDS as opposed to the majority.

Hence, the present inventors have identified a number of genes and their regulatory elements implicated in immune resistance to infection, particularly viral infection and more particularly HIV infection.

The identification of these genes and their regulatory elements involved in immune resistance to infection enables a series of novel modes of treatment as well as vaccine strategies and modes of diagnosis.

The present inventors have already determined that one or more gene s located in the region of human chromosome 22 that is syntenic to the region of mouse chromosome 15 between the loci D15Mit68 and D15Mit107 and their gene products (a polypeptide encoded by such gene, or a fragment of said polypeptide), is usable in the treatment or prevention of infection.

In this respect, it has been shown that the gene is a homologue or orthologue of one of the mouse genes listed in Table 2 which shows a significantly different level of expression in A/WySn strain of mice that fail to mount rapid antibody responses to FV infection compared to (B10.A x A/WySn)F₁ mice that produce FV-neutralizing antibodies by 14 days after infection. The present inventors have now determined that the human gene is a homologue or orthologue of a mouse gene selected from the list in Table 2 inclusively but not exclusively including *Q8CCA5 (APOL3), 2600013G09Rik (RABL4), Rac2, Card10, D230019K20Rik (KA93_Human), Q9D6D6 (Tob2), 2610019103Rik (C22orf18)* and *Tnfrsf13c (Baftr).*

Homologous and orthologous genes are genes from different species which have similar nucleotide sequences. Sequence similarity may be readily determined using computer programs known in the art such as those in the Wisconsin Package^{™} (Accelrys Inc., CA, USA). Where the observed sequence similarity is hypothesized to be because the genes share a common evolutionary origin, the genes are termed "homologous", however this term is also often used loosely to indicate merely that gene sequences are very similar. The term "orthologous" is also applied to genes from different species that are hypothesized to have evolved from a common ancestor.

Accordingly, in a first aspect, this invention provides an isolated nucleic acid encoding a gene which is a homologue or orthologue of a mouse gene selected from the list in table 2 particularly, but not exclusively, containing *Q8CCA5 (APOL3), 2600013G09Rik (RABL4), Rac2*, *Card10, D230019K20Rik (KA93_Human), Q9D6D6 (Tob2), 2610019I03Rik (C22orf18)* and *Tnfrsf13c (Baffr).*

In addition, the gene is preferably one of the following human genes which show significantly different expression levels between ESNs/EUIs and HIV-1-infected individuals upon in vitro stimulation of their PBMCs with HIV-1 antigens, or at which locus significant genetic differences are demonstrated between ESNs and HIV-1-infected individuals as groups, such as, for example, the genes *Rac2, PSCD4, Card10, and Grap2.* The most preferred of these genes is *Rac2* or *PSCD4.*

The genes recited above (*Q8CCA5 or APOL3, 2600013G09Rik or RABL4,* Rac2, *PSCD4, Card10, 0230019K20Rik or KA93_Human, Grap2, Q9D6D6* or *Tob2, 2610019I03Rik or C22orf18,* and *Tnfrsf13c or Baffr*) all encode proteins or polypeptides. One or more of genes and the proteins or polypeptides encoded by these genes (and their secondary or tertiary derivatives) must be involved in the observed immune resistance to infection.

Accordingly, the present invention also provides the use of the protein or polypeptide encoded by one or more of these genes in the treatment or prophylaxis of infection, particularly viral infection, and for their use in a person who has been diagnosed as suffering from an infection or who has been identified as having a predisposition to infection may be beneficial in the treatment or prevention of infection, respectively. Preferably, the infection is a viral infection, most preferably a retroviral infection and especially HIV infection.

Advantageously, a mixture of polypeptides according to the invention, i.e. polypeptides encoded by different genes located in the region of human chromosome 22 that is syntenic to the region of mouse chromosome 15 between the loci D15Mit68 and D15Mit107, or fragments thereof, may be used in the treatment or prevention of infection. Such a mixture is expected to be more effective in treating or preventing infection than one polypeptide on its own.

Additionally, the glycosylation, sulphonation, phosphorylation, acetylation or other addition or substitution products, homologues, splice variants, transcription variants or products derivable from the nucleonic acid sequence of the genes may be used for this purpose and hence are considered to constitute part of the present invention.

Polypeptides according to the invention are coded by genes associated with naturally occurring immune resistance against establishment of HIV infection. Hence, any molecule that mimics or facilitates the action of the polypeptides according to the invention can potentially be used as a drug to enhance a vaccine regimen or to stimulate antibody production in already infected people. Such molecules are therefore part of the present invention.

For example, since Rac2 is known to be involved in T-cell activation, and as such it is possible to make a drug which mimics or facilitates the action of the expression product of this gene in T cells, or it is possible to target the downstream signals to activate T cells. The gene *Card10* and its expression product can be used in the same way.

Furthermore, polypeptides according to the invention, and drugs that mimic or facilitate their action, may be used to induce or promote stronger immunoglobulin (Ig) response in a subject and to induce or promote class switching by using in combination with a vaccine regimen.

The present inventors have already determined that one or more genes located in the region of human chromosome 22 that is syntenic to the region of mouse chromosome 15 between the loci D15Mit68 and D15Mit107, or their encoded polypeptide products or fragments of said polypeptides, can be used in the manufacture of a medicament for the treatment or prevention of infection.

It has now been found that a surprising beneficial effect is found when the genes are selected inclusively but not exclusively from the following group: *Q8CCA5 or APOL3, 2600013G09Rik or RABL4, Rac2, PSCD4, Card 10, D230019K20Rik or KA93_Human, Grap2, Q9D6D6* or *Tob2, 2610019103Rik or C22orf18,* and *Tnfrsf13c or Baffr and their* polypeptides derived from the said genes are used.

Preferably the medicament comprising one or more of the gene products from the above genes is used for the treatment or prevention of a viral infection, such as an infection caused by a retrovirus, for example an oncovirus, a lentivirus, or a spumavirus. HTLV and BLV (bovine leukaemia virus) are examples of oncoviruses which cause leukaemia. HIV and SIV are examples of lentiviruses which cause inflammatory and wasting disease. Human spumavirus is an example of a spumavirus. Most preferably the medicament is for the treatment or prevention of HIV infection.

Where the medicament is for the prevention or prophylaxis of infection, the medicament is suitably a vaccine.

In a third aspect, the invention also provides vaccine comprising one or more polypeptides encoded by the genes or one or more isolated nucleic acid selected particularly, but not exclusively, from the following group: *Q8CCA5 or APOL3, 2600013G09Rik* or *RABL4, Rac2, PSCD4, Card 10, D230019K20Rik or KA93_Human, Grap2, Q9D6D6* or *Tob2, 2610019103Rik or C22orf18,* and *Tnfrsf13c or Baffr* and a pharmaceutically acceptable carrier. Suitable pharmaceutically acceptable carriers are well known in the art.

In a fourth aspect, the invention provides a method of treating or preventing infection comprising administering a pharmaceutically effective amount of one or more polypeptides or fragments of said polypeptides encoded by genes selected particularly but not exclusively from the following group: *Q8CCA5 or APOL3, 2600013G09Rik or RABL4, Rac2, PSCD4, Card10, D230019K20Rik or KA93_Human, Grap2, Q9D6D6* or *Tob2*, *2610019103Rik or C22orf18,* and *Tnfrsf13c or Baffr.*

As noted in the introduction, the present inventors have previously described microsatellite markers which appear to be associated with immune resistance to HIV infection in a group of ESNs and mapped these to a region of chromosome 22 that is syntenic to the area of mouse chromosome 15 containing a retrovirus resistance gene *Rfv3* (molecular identity unknown). The inventors have now identified the genes in this same region which are differentially expressed in mice that are capable or incapable of producing virus-neutralizing antibodies upon FV infection and this provides the first indication of the molecular identities responsible for early immune resistance to virus infection.

Further, the present inventors have now demonstrated that some genes in the syntenic region of human chromosome 22 are expressed higher in PBMCs of ESNs than in those of HIV-1-infected individuals upon stimulation with HIV-1 antigens. Moreover, these differences are associated with base changes in the nucleic acid sequence of their regulatory elements. These findings enable the manipulation of the mechanism of immune resistance to prevent or treat infection and for the determination of products which can be used for this purpose.. This may be done at the level of the gene product, as outlined above, at the level of the regulation of gene expression as exemplified below, or at the level of the gene itself, by gene therapy.

Hence, in a fifth aspect, the invention provides a method of treating or preventing infection comprising augmenting or inhibiting expression of one or more genes located in the region of human chromosome 22 that is syntenic to the region of mouse chromosome 15 between the loci D15Mit68 and D15Mit107.

The preferred genes are selected from the list consisting inclusively but not exclusively from the following: *Q8CCA5 or APOL3, 2600013G09Rik or RABL4. Rac2*, *PSCD4, Card10, D230019K20Rik or KA93_Human, Grap2, Q9D6D6 or Tob2, 2610019103Rik or C22orf18,* and *Tnfrsf13c or Baffr.*

If a gene as described herein shows high expression, for example, in (B10.A x A/WySN)F₁ mice relative to expression in A/WySn mice, or in humans a gene shows high expression in ESNs/EUIs relative to HIV-1-infected individuals, it may be considered as gene associated with resistance or a "resistance gene". Conversely, if a gene as described herein shows high expression in A/WySn mice relative to expression in (B10.A x A WySn)F₁ mice, or a gene shows high expression in HIV-1-infected individuals relative to ESNs/EUls, it may be considered as a gene associated with susceptibility or a "susceptibility gene". In direct gene therapy or a therapy based on the regulation of gene expression to prevent or treat viral infection, it is desirable to restore or augment expression of resistance genes but inhibit or prevent expression of susceptibility genes. It is a feature of the present invention that target genes for such therapy have been identified and that gene therapy products can be designed for these targets.

Host genetic factors influencing viral entry and replication and immune responses against retroviral infections have been extensively studied by using mouse models [38-41]. Friend mouse leukaemia virus complex (FV) is composed of replication-competent Friend mouse leukaemia helper virus (F-MuLV) and defective spleen focus-forming virus. FV induces rapid proliferation of infected erythroid progenitor cells upon inoculation into immunocompetent adult mice of susceptible strains. Persistent infection of FV associated with severe immunosuppression ultimately causes the emergence of mono- or oligoclonal expansion of leukaemia cells due to an insertional activation of a cellular transcription factor or disruption of a tumour suppressor gene. Host gene loci, *Fv1, Fv2,* and *Fv4,* that directly control the viral entry and replication in the target cells have been identified [42-45]. However, even when the host animals share the same susceptible genotypes at the above loci, the rate of disease development and progression still changes drastically depending on host genotypes at several loci that influence immune responses to FV antigens [40]. Two major histocompatibility complex (MHC) class II loci directly restrict the T helper cell recognition of the viral envelope antigen [46, 47], while a class I locus influences the production of cytokines from viral antigen-specific T-cells [48]. Another locus mapped in the MHC class lb region may affect natural killer cell functions [49, 50]. Yet another host locus that has been mapped in chromosome 15, and thus is irrelevant to MHC, strongly influences the persistence of viraemia after FV infection [40, 51-53]. Genotypes at the same non-MHC locus also influence the production of cytotoxic antibodies that modulate the expression of viral antigens on infected cell surfaces [54]. However, possible relationship between the persistence of viraemia and production of virus-neutralizing antibodies has not been directly examined. The inventors have performed linkage analyses on a mouse locus that is postulated to be connected with immune activation that may be responsible for resistance of the various mouse strains to FV and linked to their ability to respond to FV infection with virus-neutralizing antibodies. An extension of this mouse study to syntenic regions in ESN/EUI humans unexpectedly led to a demonstration of human chromosomal markers that are associated with strong immune responses to HIV-1 in HIV-uninfected individuals, as described in International patent publication no. WO 2004/035825.

The gene *Rfv3* was originally defined as a single autosomal gene that determines whether mice infected with Friend leukaemia retrovirus recovered from viraemia by 30 to 60 days after infection or not [40, 51]. This gene has been mapped to mouse chromosome 15 [52, 53], although its molecular identity is still unknown. Immune resistance against Friend retrovirus infection is also influenced by genes of mouse major histocompatibility complex (MHC), H2, which control T-lymphocyte responses to the viral envelope and gag antigens [40, 46, 49 and 55]. When tested in congenic strains, early production of virus-neutralizing antibodies was observed in mice that possessed either a resistant allele (*Rfv3^{r}*) at the *Rfv3* locus or a responder haplotype (H2^{b}) at mouse MHC, suggesting that *Rfv3* and H2 may effect the immune system through a common pathway. Moreover, mice possessing both an *Rfv3^{r}* allele and an H2^{b} haplotype showed even higher levels of virus-neutralizing antibodies and a higher frequency of IgM to IgG class switching in comparison with the H2^{a/b} mice lacking an *Rfv3^{r},* further indicating that *Rfv3,* in cooperation with H2, might regulate a T-helper cell function. This was of potential relevance to why HIV-specific IgA production, in the apparent absence of IgG, can be detected in ESNs, especially because HIV-1 antigen-specific T helper cell responsiveness and patterns of cytokine production from T cells may differ between ESN and HIV-infected individuals [14, 16, 19].

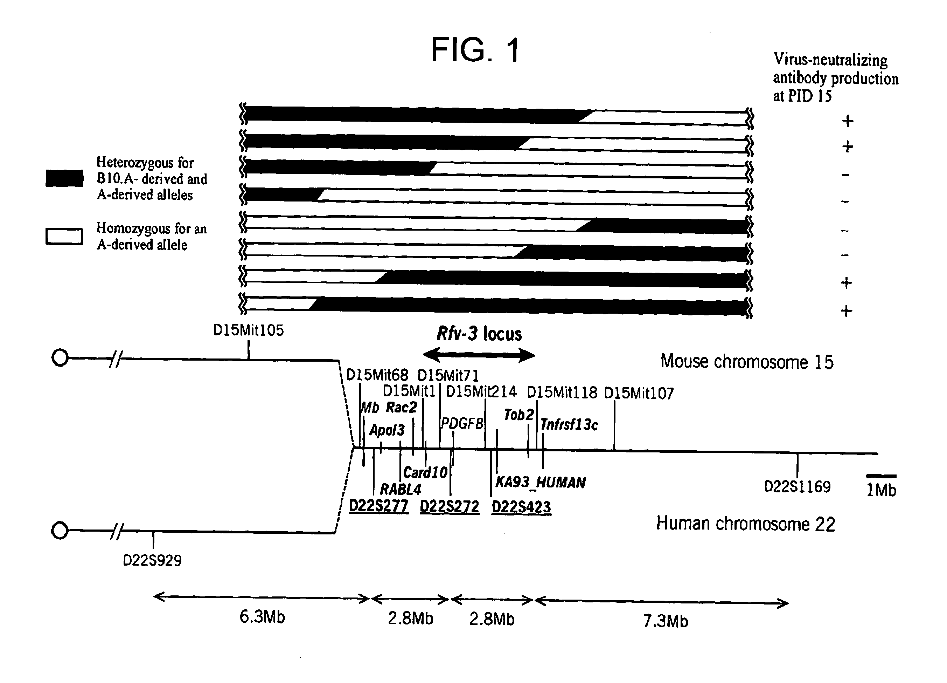

The *Rfv3* locus had been mapped in mouse chromosome 15 between the D15Mit1 and D15Mit118 loci (Fig. 1). The present inventors assembled a comprehensive list of genes and open reading frames (ORFs) located in the area surrounding the above region between the Mb (Myoglobin) and D15Mit107 loci based on the genome database information compiled in the Ensembl Genome Browser (http://www.ensembl.org/), along with accession numbers of each gene and ORF (Table 2). Two oligodeoxynucleotide probes were designed for each of the above genes and ORFs by using the Target Specifier software (CombiMatrix Corporation, Mukilteo, Washington) and synthesized on microarray chips.

The microarray chips were used to analyse levels of expression of genes located within the above region of chromosome 15 in Rfv3^{s/s} mice (that have median survival time of 40 days post infection with 15 spleen focus-forming units of Friend virus and which also lack the production of F-MuLV-neutralizing antibodies at post inoculation days (PID) 14 and 20) and *Rfv3*^{*r*/*s*} mice (that have median survival time of 70 days post infection with 15 spleen focus-forming units of Friend virus and produce F-MuLV-neutralizing antibodies at PID 20) following inoculation with Friend virus complex, as described in the example.

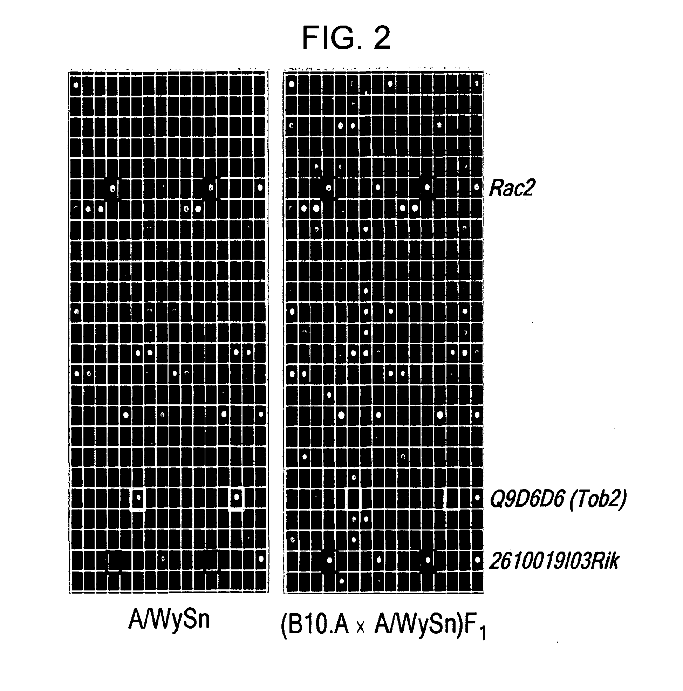

Overall levels of expression and their differences between *Rfv3*^{*s*/*s*} A/WySn and *Rfv3*^{*r*/*s*} (B10.A x A/WySn)F₁ mice of genes located within the above chromosome 15 region were largest at PID 9. An example of the resultant microarray images is shown in Fig 2. In these particular arrays, hybridization of fluorescent-labeled cRNA samples prepared from the spleen of A/WySn and (B10.A x A/WySn)F₁ mice at PID 9 were compared. Fluorescent intensities for each expressed gene were compared between the two strains. There were two microarray spots for each gene and the experiments were conducted on two mice per strain. The mean value from two microarray spots per each gene was obtained and the difference in expression between the strains of mice was considered significant if the ratio of expression was 2.9 to 3 (or more) times higher or lower on two separate occasions and with the level of expression being at least 15,000 in fluorescence intensity on at least one occasion. The expression of a gene at the level of 15,000 was considered significantly high based upon the average level of expression of all the genes on the chip being 8,992 and 5,495 for FV resistant strain, *Rfv3*^{*r*/*s*} (B10.A x A/WySn)F₁ and 6,126 and 3,868 for FV susceptible strain, *Rfv3*^{*s*/*s*} A/WySn (with the average for all four mice being 6,120). Most of the genes included in the arrays showed similar levels of expression between the two strains. Relative levels of expression of each gene at PID 9 are summarized in Table 2. Interestingly, however, there are a few genes of which the levels of expression between *Rfv3*^{*s*/*s*} A/WySn and *Rfv3^{rls}* (B10.A x A/WySn)F₁ mice at PID 9 were strikingly different.

Genes of interest revealed by the present study therefore include inclusively but not exclusively all those genes listed in Table 2 which fulfil the above criteria.

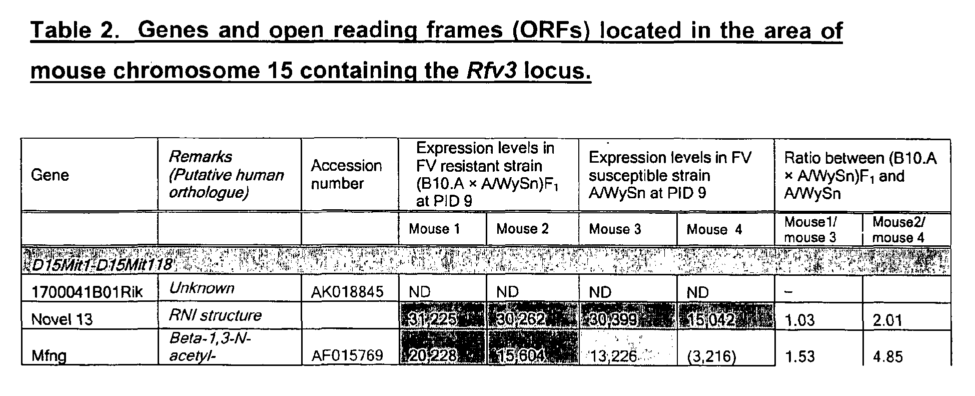

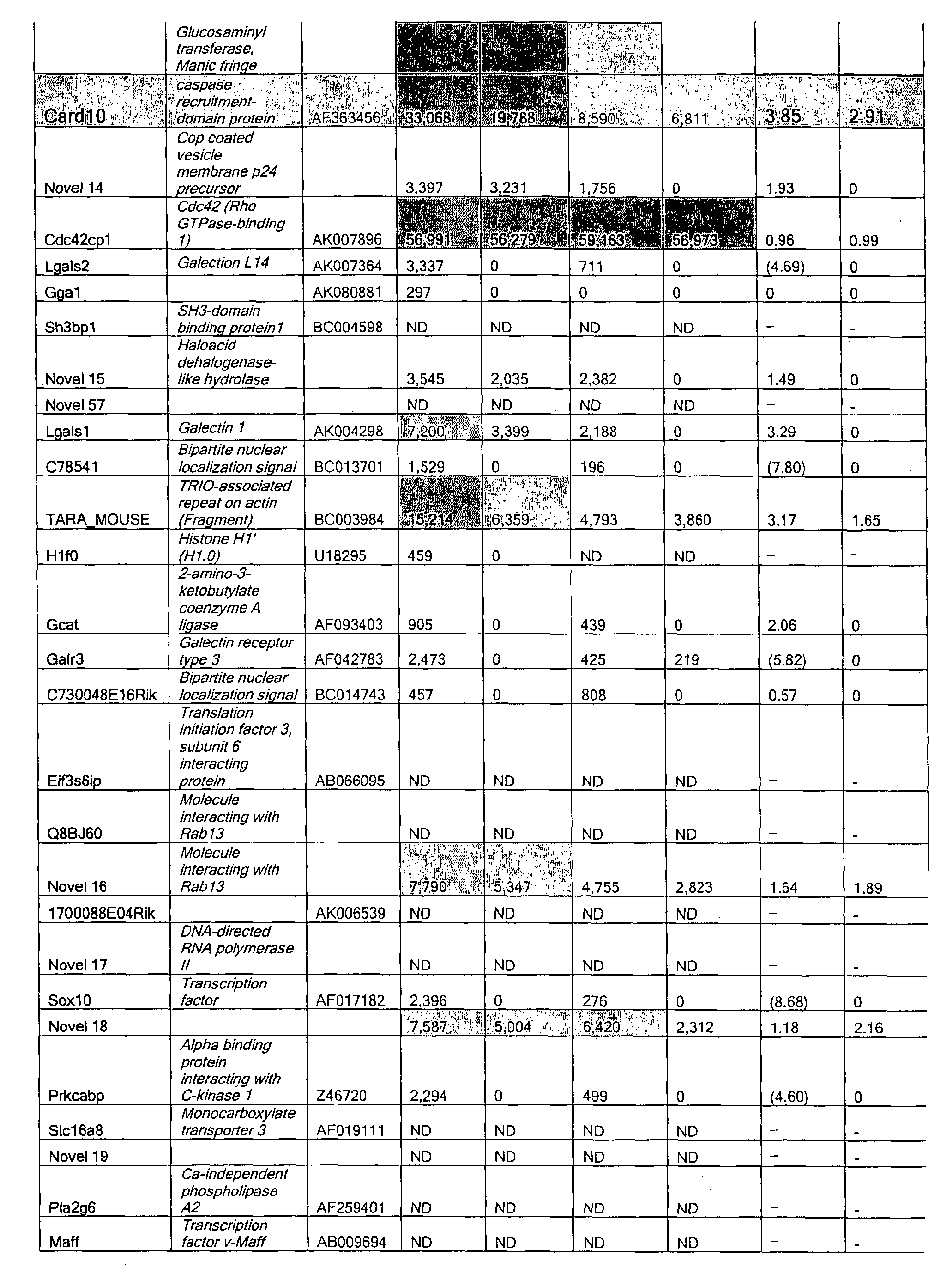

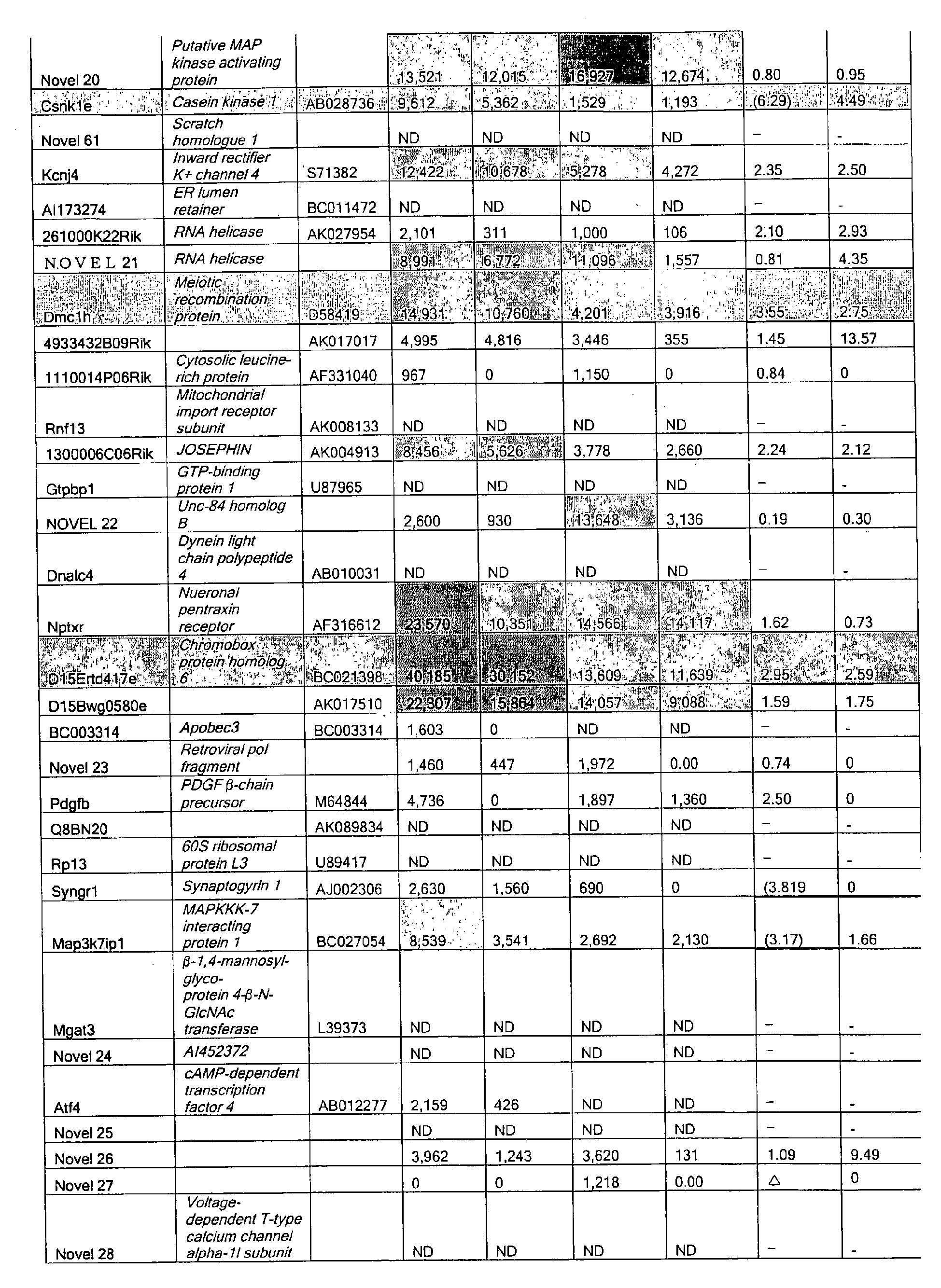

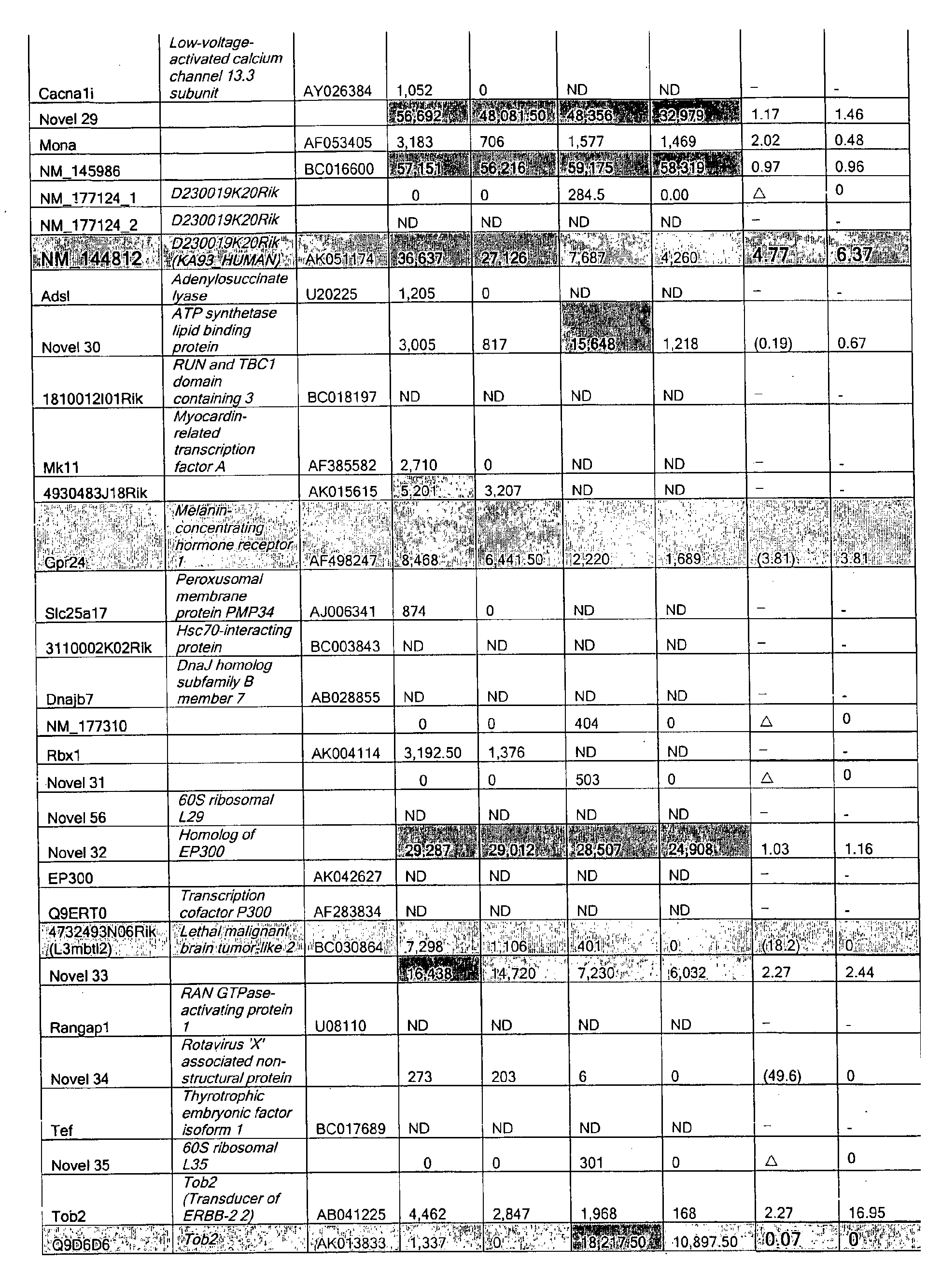

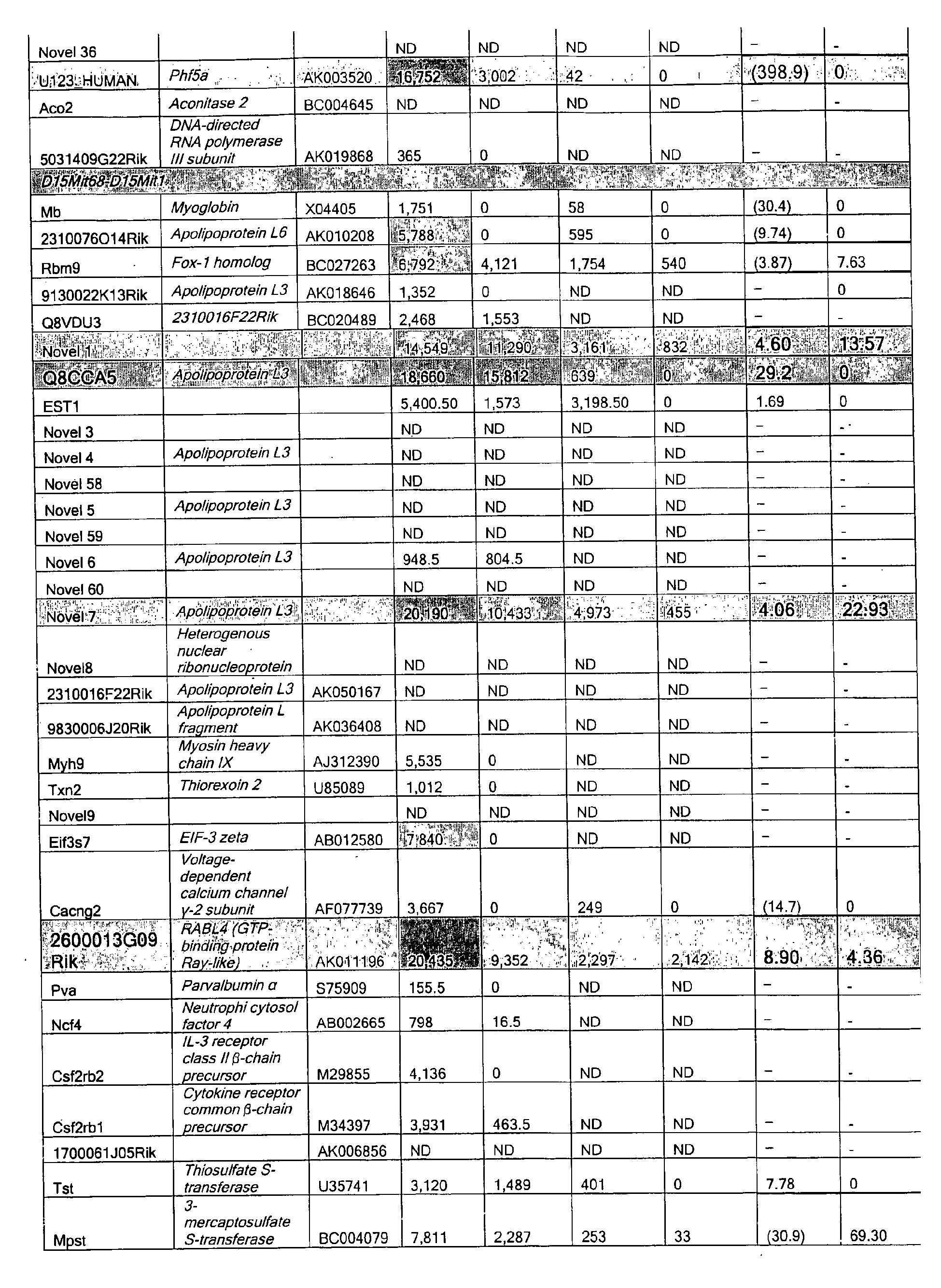

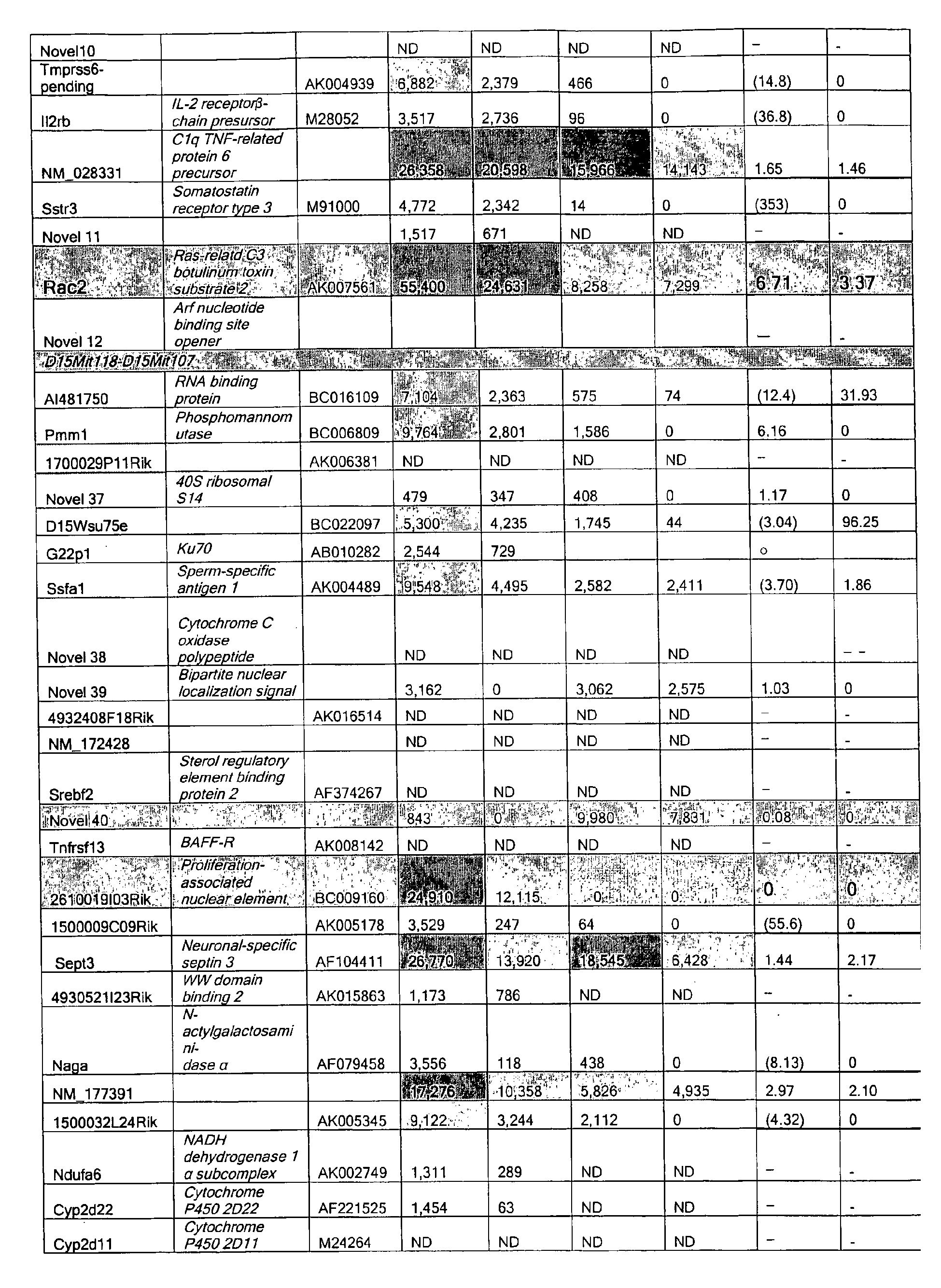

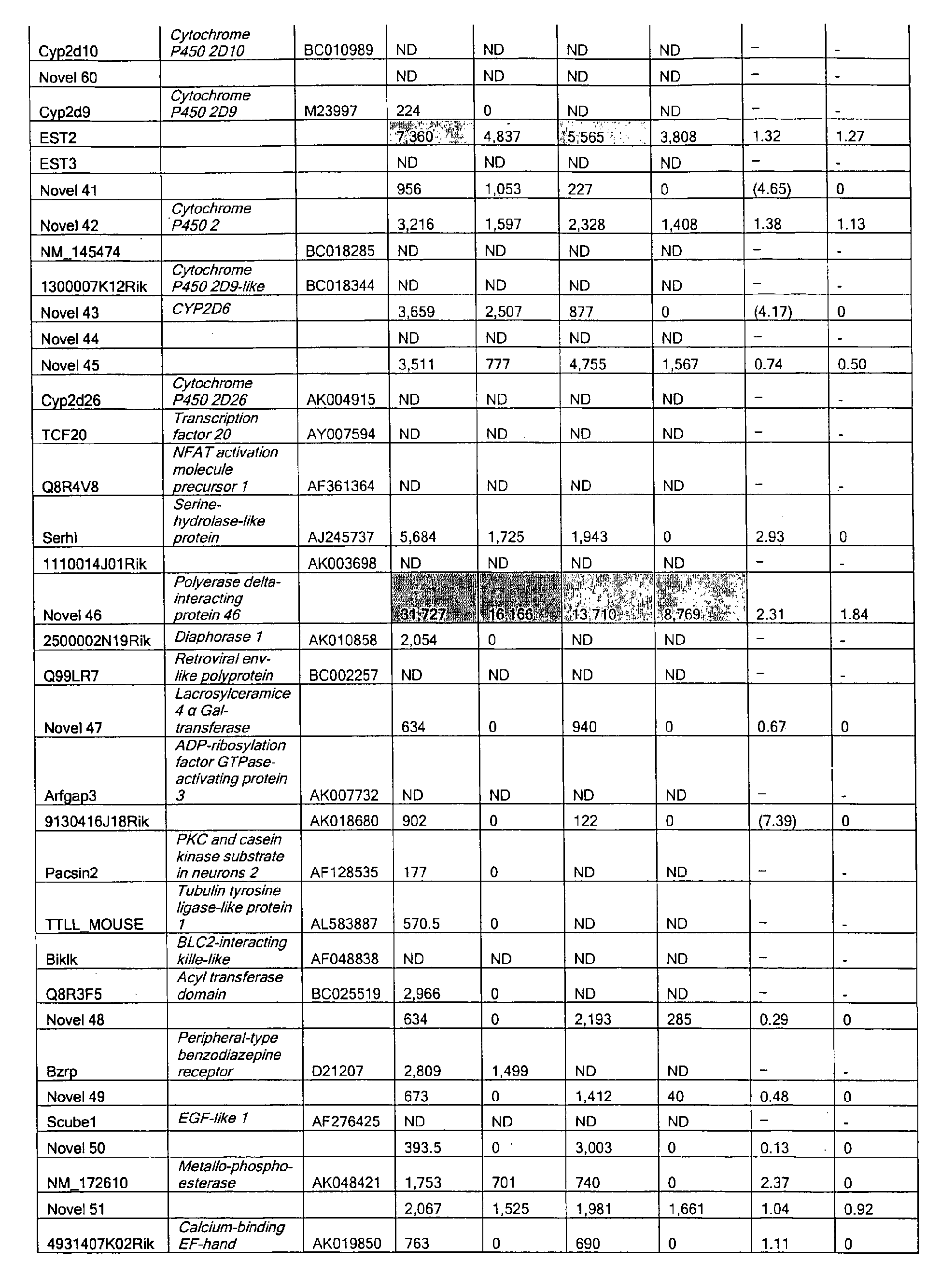

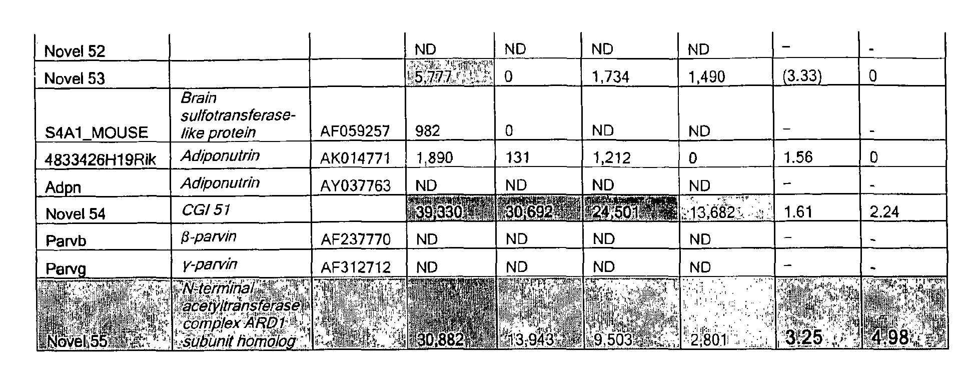

Genes selected as being of particular interest include:
- *Q8CCA5*, mouse homologue of human *Apol3* that encodes Apolipoprotein L3 inducible by TNF-α in vascular endothelial cells Vascular endothelial genes that are responsive to tumor necrosis factor-alpha in vitro are expressed in atherosclerotic lesions, including inhibitor of apoptosis protein-1, stannin, and two novel genes [56].
- *2600013G09Rik* the mouse orthologue of human *RABL4* gene,
- *Rac2* that is involved in haematopoietic cell egression from the bone marrow and neutrophil chemotaxis [57],
- *Card10* that encodes caspase recruitment-domain protein involved in NF-κB activation in T and B cells [58]. Card10 is a novel caspase recruitment domain/membrane-associated guanylate kinase family member that interacts with BCL10 and activates NF-kappa B,
- *D230019K20Rik* whose human homologue is *KA93_HUMAN,* and that is located just adjacent to the D22S423 marker at which the inventors have shown the genetic difference between the ESN/EUI and HIV-1-infected groups of individuals.
- *Q9D6D6 or Tob2,* an anti-proliferative protein [59] Tob2, a novel anti-proliferative Tob/BTG1 family member, associates with a component of the CCR4 transcriptional regulatory complex capable of binding cyclin-dependent kinases that is highly expressed in the susceptible A/WySn but not in the antibody-producing (B10.A x A/WySn)F₁ mice, and
- *2610019103Rik,* the orthologue of human *C22orf18,* that is adjacent to *Tnfrsf13c* (*Baffr*) gene encoding the BAFF-receptor in both mice and humans, and shows the patterns of expression similar to the *Tnfrsn13C* gene ([60]. Tnfrsf13c (Baffr) is misexpressed in tumors with murine leukemia virus insertions at Lvis22. Based on the previous identification of genetic markers associated with early immune resistance against repeated exposure to HIV-1 located in the region of human chromosome 22 that is syntenic to mouse chromosome 15 where the *Rfv3* gene was mapped (International patent publication no. WO 2004/035825; Fig 1), it is likely that human orthologues of one or more of the above "candidate genes" are involved in the early immune resistance against HIV-1 infection in humans.

As described, the present inventors have previously mapped genes associated with the HIV-exposed but uninfected status in a segment of human chromosome 22 that is linked with microsatellite markers *D22S277, D22S272, and D22S423* (International patent publication no. WO 2004/035825) [61]. Further, a disruption of linkage disequilibrium across the chromosome 22 loci at the *D22S276* locus, which is telomeric to the above three loci, was observed only in the group of HIV-exposed but uninfected individuals but not in the groups of HIV-infected or healthy control individuals, indicating that a possible chromosomal recombination and/or mutation might have taken place only in the ancestors of the ESN/EUI individuals at the area surrounding this locus. The above result on the disruption of linkage disequilibrium at the *D22S276* locus indicates that the putative gene that confers immune resistance against the establishment of HIV infection may exist in the region of human chromosome 22 centromeric to the *D22S276* locus.

To ensure that the current patient population used to generate current data was the same as the population used in [61], the present inventors genotyped and compared the profiles carrying out a similar analysis at allele 229 of the D22S423 locus. The number of enrollees possessing the allele 229 at the *D22S423* locus was significantly higher among the ESN than among the HIV-infected individuals (28/68 of ESNs tested were genotype 229 at D22S423 versus only 11/70 in HIV+ group); *P*= 0.0012 by Fisher).

To further narrow down the chromosomal region where the putative immune resistance gene is located, we genotyped 74 HIV-exposed but uninfected and 77 HIV-infected individuals enrolled from the same geographical region at loci of single nucleotide polymorphism (SNPs) SNP loci were selected based upon their location on chromosome 22 between the *APOL3* and *A4GALT* loci to cover the above candidate region. The segment between the *APOL3* and *A4GALT* includes the region syntenic to the segment of mouse chromosome 15 that harbors the host resistance gene, *Rfv3* [58]. In addition, two other criteria for the selection of SNP loci to be genotyped were: 1/reported frequencies of different alleles among Caucasians (SNP Browser ver. 3.0, Applied Biosystems, Foster City, CA) with each low-frequency allele expected to be found in roughly 20 to 40% of the tested individuals, and 2/ unskewed distribution of the SNP loci within the above chromosomal segment.

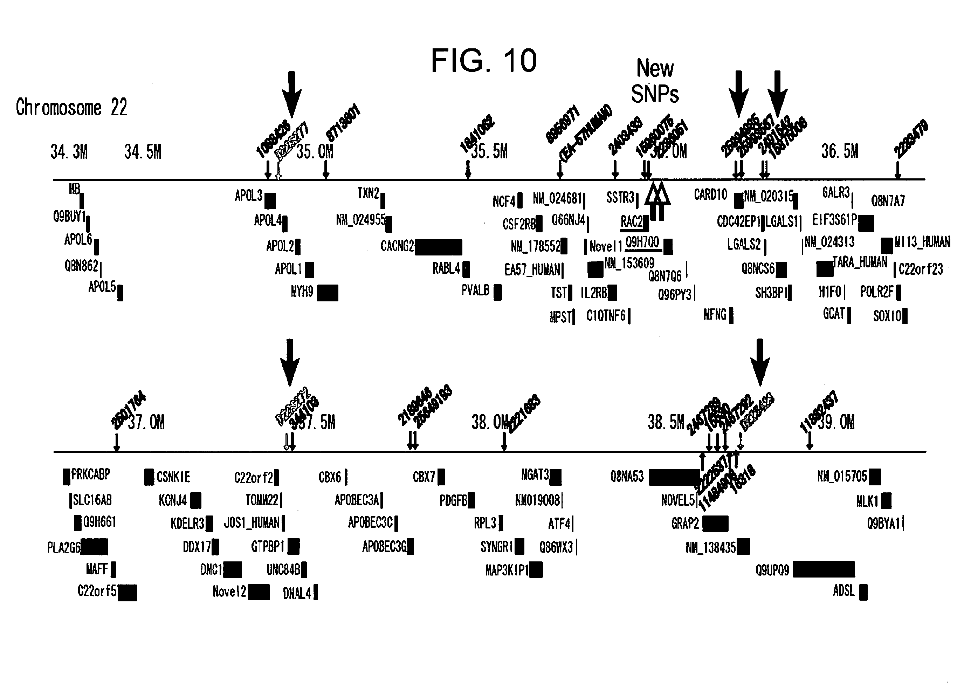

Table 3 and Figure 10 show the list of SNP loci genotyped by the present inventors:

**TABLE 3**

| **hCV ID number** | **Linked Gene** | **allele1** | **allele2** |
|---|---|---|---|
| 1088426 | *APOL3* | *A* | *T* |
| 8713601 | *MYH9* | *A* | *G* |
| 1841062 | *RABL4* | *A* | *G* |
| 8956971 | *EA57 HUMAN* | *C* | *T* |
| 25968036 | *EA57_HUMAN* (exon 1 coding) | *A* | *G* |
| 2403433 | *IL2RB* | *G* | *T* |
| 2403368 | *C1QTNF6* | *C* | *G* |
| 15960075 | *RA C2* | *A* | *G* |
| 2236051 | *RA C2* | *C* | *T* |
| 27466802 | *CARD10* | *A* | *G* |
| 8957740 | *CARD10* | *C* | *T* |
| 25994985 | *CARD10* | *C* | *T* |
| 25993567 | *CARD10* | *C* | *T* |
| 2491542 | *CDC42EP1* | *A* | *G* |
| 15875008 | *LGALS2* | *A* | *G* |
| 2233479 | *POLR2F* | *C* | *T* |
| 2501764 | *MAFF* | *A* | *T* |
| 344103 | *GTPBP1* | *G* | *C* |
| 2189646 | *APOBEC3G* (exon4 coding) | *A* | *G* |
| 25649193 | *APOBEC3G* (exon6 coding) | *C* | *G* |
| 2221682 | *RPL3* | *G* | *A* |
| 2222537 | *GRAP2* promoter | *A* | *G* |
| 2222563 | *GRAP2* (intron 1) | *A* | *G* |
| 2467289 | *GRAP2* (intron 2) | *A* | *G* |
| 15530 | *GRAP2* (intron 3) | *A* | *G* |
| 2467292 | *GRAP2* (intron 3) | *G* | *T* |
| 11484908 | *GRAP2* (exon8 coding) | *C* | *T* |
| 16318 | *GRAP2* (3' intron) | *C* | *T* |
| 11882437 | *Q9UP9Q (TNRC6B)* | *A* | *G* |
| 224082 | *NOVEL10 (LOC63929)* | *C* | *T* |
| 2497323 | *TOB2* | *G* | *A* |
| 2481122 | *NM 024821 (FLJ22349)* | *C* | *G* |
| 2189968 | *BAFF-R* | *A* | *G* |
| 2189972 | *C22orf18* | *A* | *G* |
| 2468720 | *TCF20* | *A* | *T* |
| 2986155 | *NM 170698 (dJ222E13.2)* | *A* | *G* |
| 1150511 | *A4GALT* | *C* | *G* |

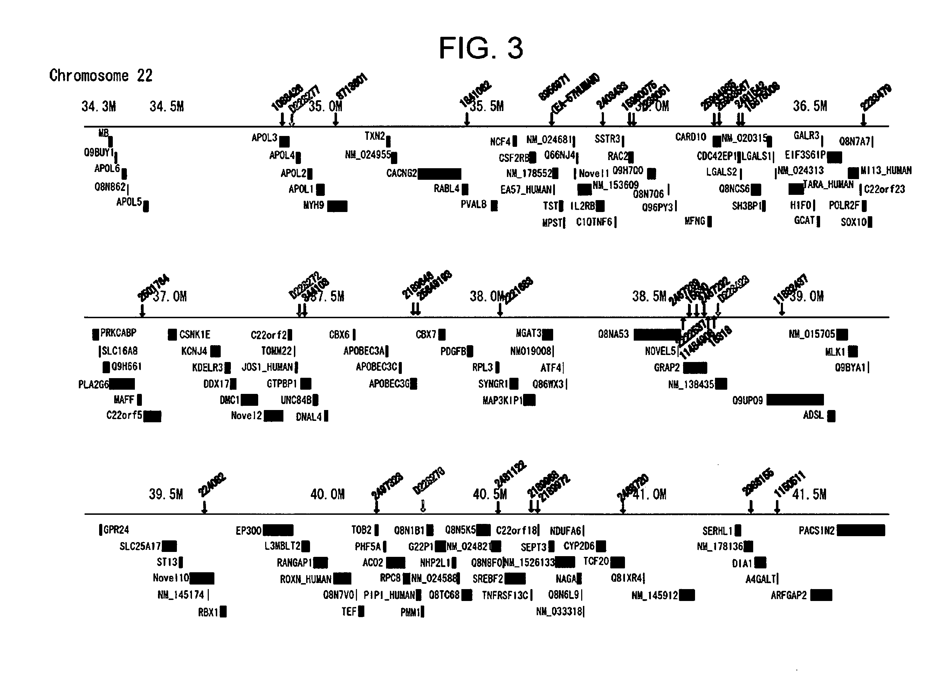

Locations of these genetic loci are shown in Figure 3 where physical map positions of the relevant microsatellite markers are shown in blue, and physical locations of the tested SNP loci are shown in brown (two SNP loci linked to the *Card10* locus are omitted from the Figure because they are close to the other two loci shown). Known open reading frames are also included in this Figure. Fluorescence-labeled sets of appropriate primers and probes were purchased from Applied Biosystems and genotyping was performed by Taqman assays with the Prism 7700 real-time PCR system according to the manufacturer's instructions.

The numbers of individuals possessing the indicated allele 1 are significantly higher among the ESNs under a dominant gene hypothesis at the *Card10, CDC42EP1,* and *GRAP2* loci, and the most highly significant difference between the ESN and HIV-infected individuals was observed at the CDC42EP1 locus (P = 0.0043), as described in the Example 3 below.

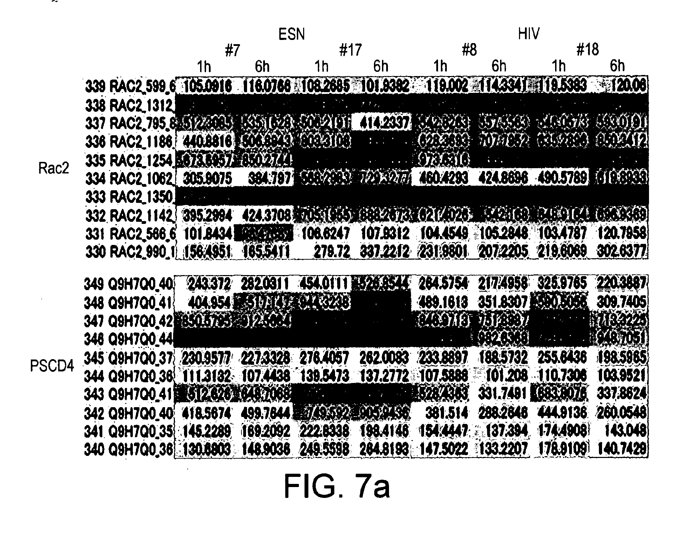

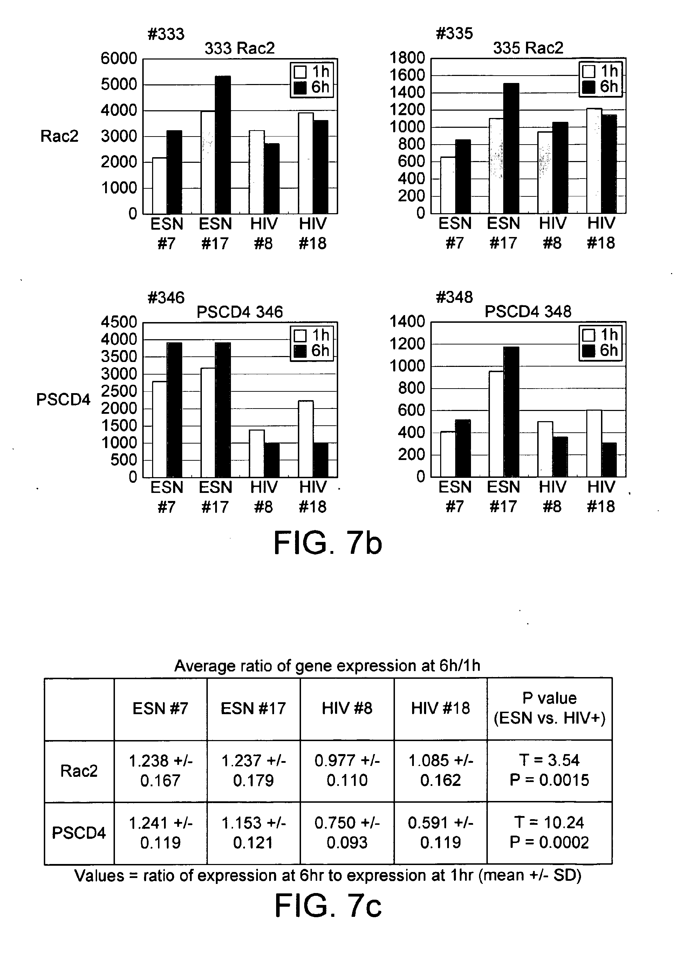

In addition to the genotyping at the above SNP loci, expression levels of all the genes located in the above candidate region was compared between the HIV-exposed but uninfected and HIV-infected individuals. To detect the possible changes in the expression of host genes that are associated with observed immune resistance against HIV infection without being biased by host factors other than HIV resistance and environmental factors, we stimulated peripheral blood mononuclear cells prepared from each examined individuals with the mixture of promiscuous HIV-1 antigens, and prepared total RNA before and after the antigenic stimulation. Changes in gene expression were then compared between peripheral blood mononuclear cells of each single individual before and after the antigenic stimulation at 1 hour and at 6hours. As the expression of the tested genes were not significantly different between the cells before stimulation and those at 1 hour after the stimulation, all comparisons of expression in response to antigenic stimulation was carried out at 1 hour and at 6hours post stimulation. Of the genes investigated, two genes RAC2 and Q9H7Q0 (PSCD4) showed an increase in expression levels at 6 hours after the stimulation in ESNs but not in HIV+ population. All other tested genes did NOT show a significant increase. The levels of increase found are about 20%, and are consistent, as shown in Fig. 7. The colours give an indication of fluorescence levels: deep blue <100, blue: 100-200, light blue: 200-250, light pink: 250-500, pink: 500-1000, red: 1000-2500, deep red: over 2500 >.

Microarray analyses were performed as described for the expression of mouse genes above.

Soon after the culture for the antigenic stimulation, PBMCs were treated with RNAlater (Ambion, Inc.). Total RNA was extracted from PBMCs by using the TRlzol reagent (Invitrogen Life Technologies, Carlsbad, California) according to the manufacturer's instructions. Complementary DNA (cDNA) was produced from total RNA in the presence of RNase inhibitor (Promega Corporation, Madison, Wisconsin) by using the T7-oligo (dT) 24 primer (5'-GCCCAGTGAATTGTAATACGACTCACTATAGGGAGGCGGTTTTTTTTTTT TTTTTTTTTTTTT-3', PROLIGO Japan, Kyoto, Japan) and SuperScript II reverse transcriptase (Invitrogen) according to the manufacturer's instructions. Resultant cDNA was purified with PCR purification kit (QIAGEN, K. K., Tokyo, Japan). Biotinylated cRNA was prepared by using Bio-16-UTP (Enzo Life Sciences, Inc., Farmingdale, New York) and MEGAscript transcription kit (Ambion, Inc., Austin, Texas), and resultant cRNA was purified by using RNeasy kit (QIAGEN, K. K.).

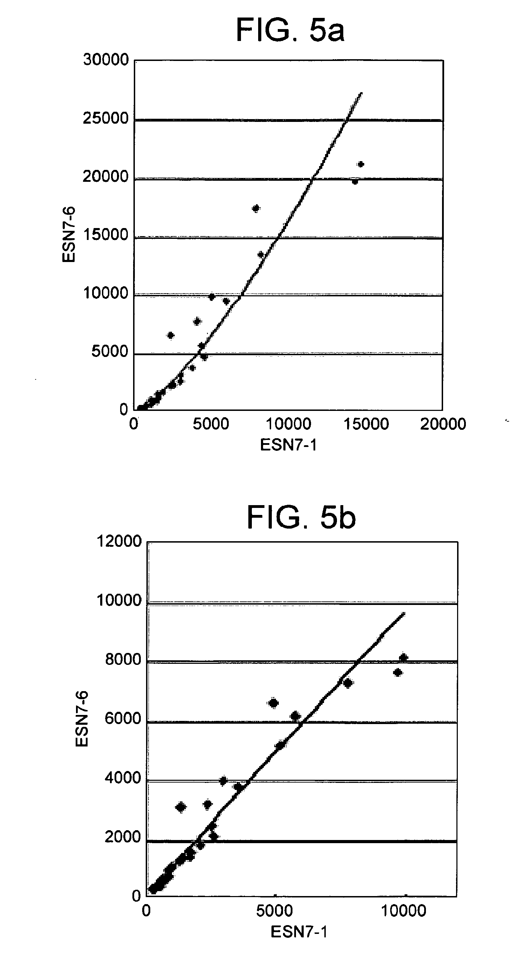

Accordingly, in a further aspect, the invention provides the identification of two genes, *RAC2* and *PSCD4* (also known and listed in Figure 3 as Q9H7Q0_*human*). The present inventors observed apparent increases in the expression of other genes in the previous analyses, but only two, RAC2 and PSCD4 have remained significant after standardization for expression levels of GAPDH (see Figure 5) in all experiments. These two genes *RAC2* and *PSCD4* were always expressed higher after antigenic stimulation of peripheral blood mononuclear cells from ESN individuals, while the expression of these genes in the cells prepared from HIV-infected individuals was unchanged. Studying expression of these genes and the expression products may be useful in further elucidating the pathology of HIV infection and, moreover, in the design of anti-HIV therapies and vaccines. In this respect, the present invention also provides polypeptides as hereinbefore described where the polypeptide is produced by synthetic means, such as using a clone, using an *in vitro* synthesis method or a protein synthesizer.

The present invention also provides isolated nucleic acids encoding RAC2 and *PSCD4* for use in medicine. These nucleic acids are also usable in the preparation of a vaccine for the prophylaxis of infection, such as viral infection and especially HIV infection.

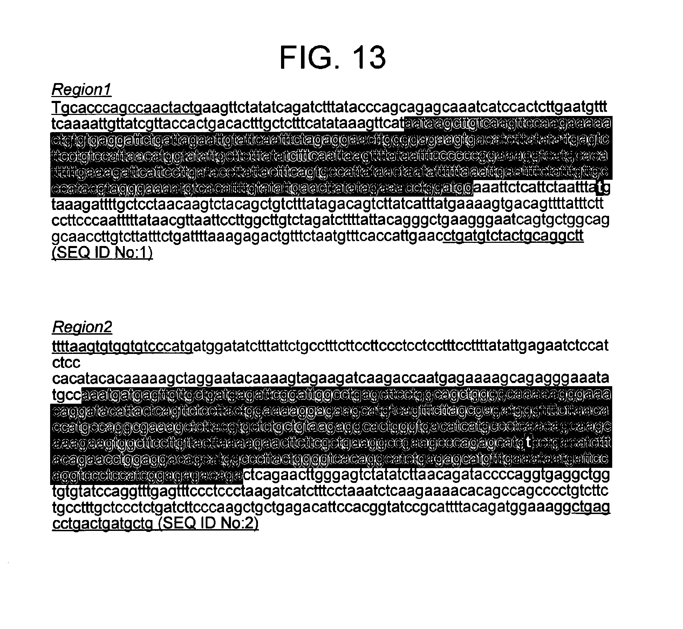

Additionally, the invention provides oligo- or polynucleotides encoded by SEQ Id No: 1 or by SEQ ID No: 2 of Figure 13, their fragments, or modified oligo- or polynucleotide with base substitutions. These sequences are involved in the regulation of the expression of Rac2 and *PSCD4* genes and include base substitutions or polymorphisms that are different between ESNs and HIV-1-infected individuals. The sequences listed are 'enhancers' which regulate the expression of multiple genes. By using enhancers, it is possible to induce the expression of endogenous genes, without directly delivering a gene of interest by gene therapy. The information on enhancers can be used to design low molecular weight drugs that bind to the target sequence and thereby modify the expression of target genes. The present invention therefore also provides for the use of these enhancers in medicine.

Accordingly, the invention also provides a method of treating or preventing infection, the method comprising administering a pharmaceutically effective amount of one or more chemical compound, synthetic oligonucleotide such as siRNA, or polypeptide binding to nucleic acid as hereinbefore described or functional fragments of said regulatory element of the genes to an individual in need of such treatment.

The invention also includes a method of treating or preventing infection, the method comprising augmenting or inhibiting expression of one or more genes encoding a polypeptide as hereinbefore described in an individual in need of such treatment.

In a further aspect, the invention also provides the use of a polypeptide as hereinbefore described or antibody raised thereto in a method of screening of compounds for functional homologues of said polypeptides.

The present invention also provides a method of determining disease progression by being able to identify the mechanism and pathway of viral infection and its resistance. Thus the invention also provides a method and compounds which can be used to modify the disease progression as well as to determine an appropriate course of treatment in an individual.

Embodiments of the invention will now be described, by way of example only, with reference to the following examples as illustrated by the appended drawings of which:-
Figure 1 is a diagrammatic presentation of the order of and distance between SSLP or microsatellite markers and homologous genes located within the syntenic region of mouse chromosome 15 and human chromosome 22;
Figure 2 is a comparison of microarray images following hybridization of fluorescent labelled cRNA sampled prepared from the spleen of A/WySn and (B10.A x A/WySn)F₁ mice at PID 9;
Figure 3 is a diagrammatic presentation of physical map positions of the relevant microsatellite markers (blue), examined single nucleotide polymorphism (SNP) loci (brown), and known genes and open reading frames (squares);
Figure 4 is a table showing the expression of the housekeeping genes beta-actin and GAPDH in human PBMCs;
Figure 5 is a pair of graphs showing the correlation between detected expression levels of GAPDH before and after standardization;
Figure 6 is a table showing the expression of representative cytokine genes at 1 or 6 hours post HIV antigen stimulation in ESNs and HIV infected individuals;
Figure 7 is a table showing *Rac2* and *PSCD4* gene expression at 1 or 6 hours post HIV antigen stimulation in ESNs and HIV infected individuals;
Figure 8 shows the distribution of linkage disequilibrium among the ESN individuals. □: 0.01 ≤ *P* <0.05; □: 0.001 ≤ *P* <0.01; ■: 0.0001 ≤ *P* <0.001; ■: 0.00001 ≤*P* <0.0001; ■: *P* <0.00001;
Figure 9 shows the distribution of linkage disequilibrium among the HIV-infected individuals. □: 0.01 ≤*P* <0.05; □: 0.001 ≤*P* <0.01; ■: 0.0001 ≤*P* <0.001; ■: 0.00001 ≤*P* <0.0001; ■: *P* <0.00001;
Figure 10 is a gene map showing the relationship between the SNPs where the genotype frequencies are different between ESNs and infected individuals, the genes in which the expression levels are different between the ESN and HIV-1-infected individuals after HIV antigen stimulation, and the location of new SNPs in their regulatory element;
Figure 11 is a printout showing areas of sequence homologies between humans and mice in the *Rac2-PSCD4* region;
Figure 12 shows sequence homology between humans and mice in the Region 2 near the *PSCD4* gene;
Figure 13 shows the nucleic acid sequence and observed SNP of the genomic sequences in the Regions 1 and 2 with appropriate primers, and

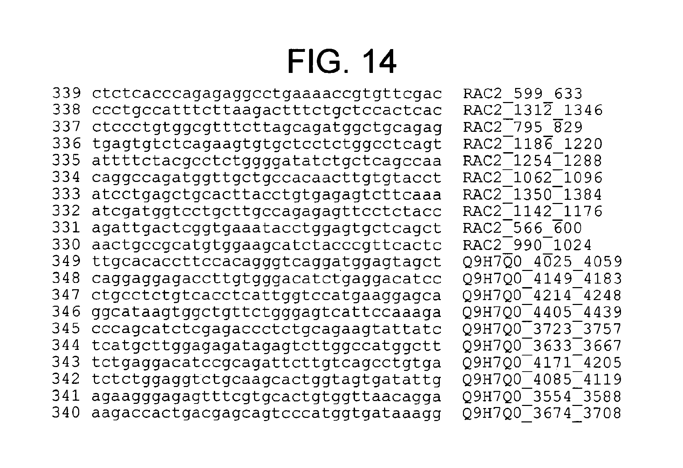

Figure 14 shows a list of the probe sequences for RAC 2 and Q9H7Q0 (PSCD4) genes.

### Example 1

### Methods

*Rfv3*^{*s*/*s*} A/WySn mice that lack the production of F-MuLV-neutralizing antibodies at post-inoculation days (PID) 14 and 20 and *Rfv3*^{*r*/*s*} (B10.A x A/WySn)F₁ mice that produce F-MuLV-neutralizing antibodies by PID 14 were inoculated with 150 spleen focus-forming units (SFFU) of Friend virus complex (FV), and the infected mice were killed at PIDs 5, 9, and 13. Control mice were not inoculated with FV. Immediately after the mice were killed, the spleen was removed from each mouse and frozen by pressing between two blocks of dry ice. Total RNA was extracted by using the TRlzol reagent (Invitrogen Life Technologies, Carlsbad, California) according to the manufacturer's instructions. Complementary DNA (cDNA) was produced from total RNA in the presence of RNase inhibitor (Promega Corporation, Madison, Wisconsin) by using the T7-oligo (dT) 24 primer (5'-GCCCAGTGAATTGTAATACGACTCACTATAGGGAGGCGGTTTTTTTTTTT TTTTTTTTTTTTT-3', PROLIGO Japan, Kyoto, Japan) and SuperScript II reverse transcriptase (Invitrogen) according to the manufacturer's instructions. Resultant cDNA was purified with PCR purification kit (QIAGEN, K. K., Tokyo, Japan). Biotinylated cRNA was prepared by using Bio-16-UTP (Enzo Life Sciences, Inc., Farmingdale, New York) and MEGAscript transcription kit (Ambion, Inc., Austin, Texas), and resultant cRNA was purified by using RNeasy kit (QIAGEN, K. K.).

The *Rfv3* locus had been mapped in mouse chromosome 15 between the D15Mit68 and D15Mit107 loci (Fig. 1). A comprehensive list of genes and open reading frames (ORFs) located in the area covering and surrounding the above region between the *Mb* (Myoglobin) and D15Mit107 loci was assembled based on the genome database information compiled in the Ensembl Genome Browser (http://www.ensembl.org/), along with accession numbers of each gene and ORF (Table 2). Two oligodeoxynucleotide probes were designed for each of the above genes and ORFs by using the Target Specifier software (CombiMatrix Corporation, Mukilteo, Washington) and synthesized on microarray chips. After the synthesis of the probes and deprotection, microarray chips were prehybridized with 10 ng/µl poly-dA and 100 ng/µl salmon sperm DNA, and biotin-conjugated cRNA samples were hybridized at 45°C, overnight after being treated at 95 °C, 20 min in acetate buffer. After hybridization, microarray chips were washed, blocked with 1% bovine serum albumin and 0.1% Tween-20, and then incubated with Cy3-conjugated streptavidin (Amersham Biosciences Corp., Piscataway, New Jersey). After vigorous washing the fluorescence image of each microarray was scanned by a GenePix scanner (Molecular Devices Corporation, Union City, California), and analyzed by using the Microarray Imager software (CombiMatrix Corporation). All probes were placed in duplicate on each single chip, and bases 8-46 (aggtctgtgtgatcatctttgaccatatagattgcctct) and 244-280 (gaacccactaagatcaaatagggtgatgctggttctg) of the GAPDH gene were included as control probes for a representative house-keeping gene.

### Results

Overall levels of expression and their differences between A/WySn and (B10.A x A/WySn) F1 mice of genes located within the above chromosome 15 region were largest at PID 9. An example of the resultant microarray images is shown in Fig. 2. In these particular arrays, hybridization of fluorescent-labeled cRNA samples prepared from the spleen of A/WySn and (B10.A x A/WySn) F1 mice at PID 9 were compared. Fluorescent intensities for each expressed gene were compared between the two strains, and most of the genes included in the arrays showed similar levels of expression between the two strains. Relative levels of expression of each gene at PID 9 are summarized in Table 2. Interestingly, however, there are a few genes of which the levels of expression between *Rfv3*^{*s*/*s*} A/WySn and *Rfv3*^{*r*/*s*} (B10.A × A/WySn) F1 mice at PID 9 were strikingly different. These include:
- *Q8CCA5*, mouse homologue of human *Apol3* that encodes Apolipoprotein L3 inducible by TNF-α in vascular endothelial cells ,
- *2600013G09Rik,* the mouse orthologue of human *RABL4* gene,
- *Rac2* that is involved in haematopoietic cell egression from the bone marrow and neutrophil chemotaxis [56]*Card10* that encodes caspase recruitment-domain protein involved in NF-κB activation in T and B cells ,
- *D230019K20Rik* whose human homologue is *KA93_HUMAN,* and that is located just adjacent to the D22S423 marker at which we have shown the genetic difference between the HIV-1-exposed but uninfected and HIV-1-infected groups of individuals,
- *Q9D6D6 or Tob2,* an anti-proliferative protein that is highly expressed in the susceptible A/WySn but not in the antibody-producing (B10.A × A/WySn)F₁ mice, and
- *2610019I03Rik,* the orthologue of human *C22orf18,* that is adjacent to *Tnfrsf13c (Baffr)* gene encoding the BAFF-receptor in both mice and humans, and shows the patterns of expression similar to the *Tnfrsn13C* gene [57]

### Conclusions

Based on the previous identification of genetic markers associated with early immune resistance against repeated exposure to HIV-1 located in the region of human chromosome 22 that is syntenic to mouse chromosome 15 where the *Rfv3* gene was mapped (International patent publication no. WO 2003/035825; Fig. 1), it is likely that human orthologues of the above "candidate genes" are involved in the early immune resistance against HIV-1 infection in humans.

### Example 2

In addition to the above analyses on the expression of mouse genes in FV-infected, resistant and susceptible animals, the inventors have also generated HUMAN microarray data.

### Methods

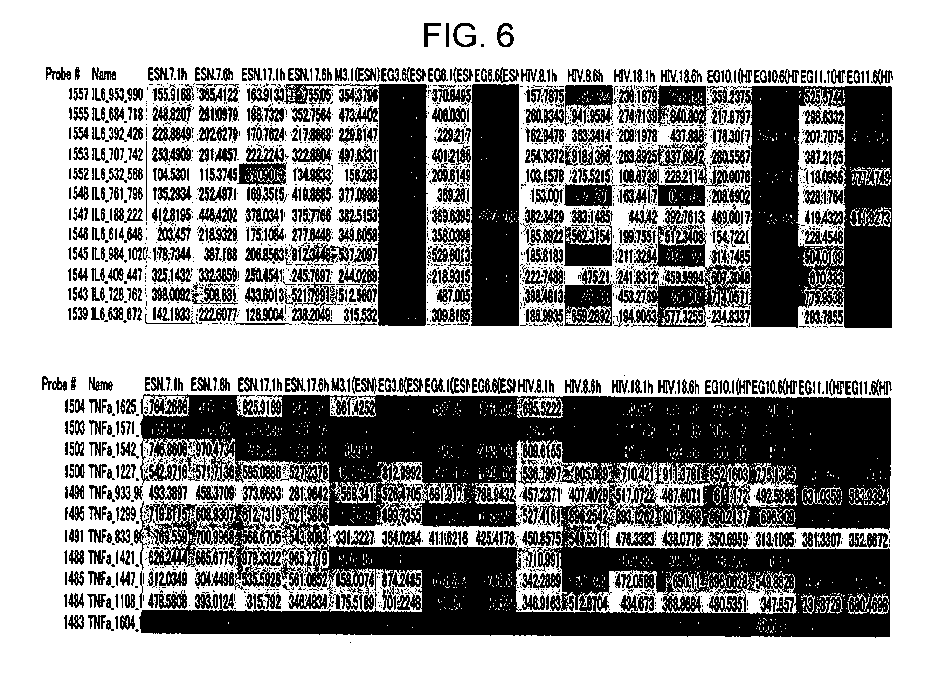

Expression levels of all the genes located in the above candidate region was compared between the HIV-exposed but uninfected and HIV-infected individuals. Our previous analyses have shown that peripheral blood mononuclear cells prepared from HIV-exposed but uninfected individuals produce significantly larger amount of IFN-y than those from HIV-exposed individuals upon stimulation with a mixture of HIV-1 Env and Gag antigens [14, 16, 57, 58, 61] Patterns of gene expression between individuals may change based on their age, sex, time-point of sample collection in a year or even in a day, and environmental factors including infectious agents, allergens, and food. To detect the possible changes in the expression of host genes that are associated with observed immune resistance against HIV infection without being biased by the above host and environmental factors, we stimulated peripheral blood mononuclear cells prepared from each examined individuals with the mixture of promiscuous HIV-1 antigens (a pool of six synthetic peptides from gag of HIV-1 at 2.5 µM final concentration plus a pool of five synthetic peptides from the gp160 envelope of HIV-1 at 2.5 µM final concentration) as described previously [14, 61], and prepared total RNA before and after the antigenic stimulation as described for Example 1. Changes in gene expression were then compared between peripheral blood mononuclear cells of each single individual before and after the antigenic stimulation. Microarray analyses were performed as described for the expression of mouse genes in Example 1 using CostomArray 12K (CombiMatrix Corporation, Mukilteo, Washington). Two to 10 specific probes were designed for each of the genes enlisted in Figure 3, and each probe was placed in at least 6 different areas in each microarray. Data obtained were standardized between individuals and between different time-points after the antigenic stimulation based on the levels of expression of two house-keeping genes, *GAPHD* and *β-actin* using a Loess equation (described in the Affy Package of Bioconductor; http://www.bioconductor.org/) as described below. Probes for the human IL-2, IFN-γ, TGF-β1, TNF-α, IL-4, IL-5, IL-6, IL-10, CD69, CD25 (IL-2Rα), CCR7, CCR5, CCR4, CCR8, CX3CR1, CCR2, SDF1, IFN-α, IFN-β, CCL3L1, IRF-9, STAT1, STAT2, Jak1, Tyk2, IFN-αR1, IFN-αR2 were also included in each microarray as positive controls. Portion of the cytokine data is shown in Figure 6.

### Results

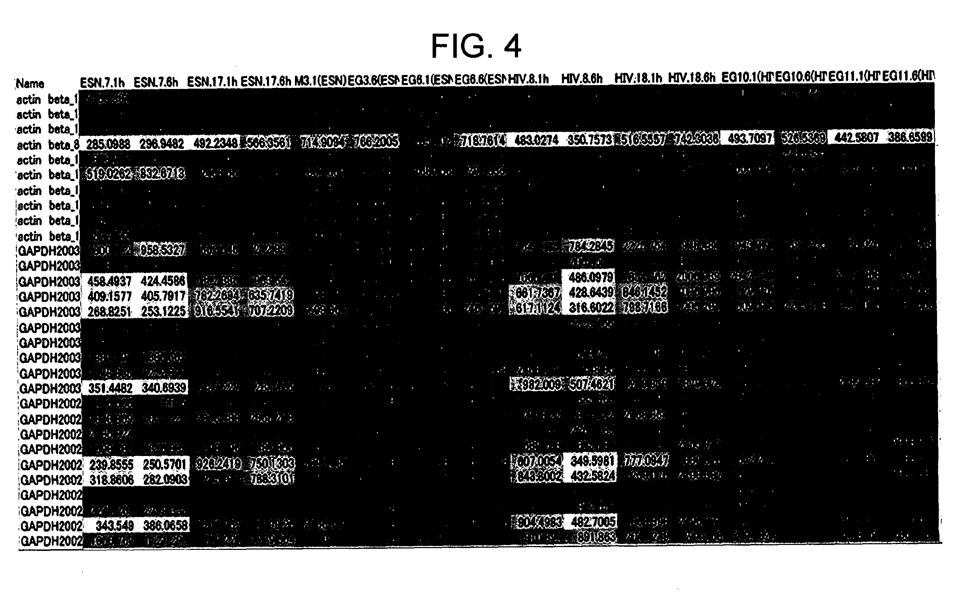

The data shown in Figure 4 are examples of the levels of expression of the housekeeping genes, β-actin and GAPDH. Samples were taken from 4 ESN (ESN7, ESN17, M3, and EG6) and 4 HIV-infected individuals (HIV8, HIV18, EG10, and EG11) at 1 (1 h) and 6 hours (6h) after the antigenic stimulation. Binding of fluorescent-labeled cRNAs to different probes (actin_beta1, actin_beta8, GAPDH2002, and GAPDH2003) at separate spots on the microarray were measured with an array data scanner (GenePix4000B, Molecular Devices Corporation, Sunnyvale, California) and quantified with appropriate software (Microarray Imager, CombiMatrix Corporation, Mukilteo, Washington). Obtained data were then standardized by using the Loess equation and are shown in Figure 5. In Figure 5, the left panel (Figure 5a) shows the non-linear correlation between the detected expression levels of GAPDH with different probes between peripheral blood mononuclear cells from ESN7 at 1 hour and 6 hours after the antigenic stimulation, and the right panel (Figure 5b) shows a linear correlation after the standardization.

The expression of the genes tested was not significantly different between the cells before the antigenic stimulation and 1 hour after the stimulation; however, dramatic changes in the expression of some genes were observed at 6 hours after the stimulation. Therefore, patterns of gene expression were compared between cells of 1 and 6 hours after the antigenic stimulation in the following analyses. Importantly and as expected, the induction of multiple cytokine genes including IL-6 and TNF-α upon the antigenic stimulation was observed even when peripheral blood mononuclear cells prepared from HIV-1-infected individuals were used, and these changes were not unique to ESNs but rather induced by antigenic stimulation in both ESNs and HIV+ individuals. The expression levels of these cytokines were often higher in the HIV-infected than in the ESN individuals at 6 hours after the antigenic stimulation, indicating that the observed differences in the gene expression levels were not simply due to the HIV-induced depletion of T cells or changes in T-cell subset compositions.. See Figure 6.

In Figures 6 and 7, probe names indicate their sequences: for example, IL_6_953_990 means that the probe contains the nucleic acid sequence of human IL-6 from base No. 953 to 990.

Among all the genes tested for their expression, two genes, *Rac2* and *PSCD4* (also known and listed in Fig. 3 as Q9H7Q0-*Human*), were always expressed higher after the antigenic stimulation of peripheral blood mononuclear cells from ESN individuals, but the expression of these genes in the cells prepared from HIV-infected individuals were unchanged or even became lower after 6 hours of antigenic stimulation. See Figure 7a and 7b. The changes shown in Figure 7 were confirmed in 4 separate ESN and 4 HIV-infected individuals using the microarray assays.

Ratios of expression levels at 1 h and 6h after the antigenic stimulation were calculated for the probes with which the expression level at 1 h was >250. Expression levels <250 suggested that the probe used reacted poorly. Averages of the 1 h/6h ratios in ESNs were consistently higher that in HIV+ individuals as seen in Figure 7c.

In addition to gene SNP analysis real time PCR was used to confirm the increased expression in Rac2 and PSCD4 in response to HIV specific antigen stimulation.

### Conclusions

We have shown that *Rac2* and *CSCD4* are induced only in PBMCs of ESNs but not in those of HIV-1-infected individuals upon HIV-1 antigenic stimulation. *Rac2* is a member of the RAS superfamily of small GTP-binding proteins, and is selectively expressed in type 1 helper T lymphocytes (Th1) in mice Rac2 induces the IFN-y promoter through cooperative interaction of the NF-κB and p38 MAP kinase pathways. Since we have shown that T cells from ESN individuals produce higher levels of IFN-γ than those from HIV-infected or healthy control individuals upon stimulation with HIV-1 antigens (mentioned above), the observed higher expression of *Rac2* upon HIV antigenic stimulation is likely to explain the higher levels of IFN-γ production in ESN individuals. *PSCD4* may also be directly involved in the immune resistance of ESN individuals, because this gene is 69% identical to the *PSCD1,* which is know to be expressed high in natural killer and T lymphocytes [59]

### Example 3

SNPs genotyping analyses also indicated that genes located near the *Rac2* and *PSCD4* are involved in the immune resistance phenotype of the ESN individuals. As shown in Table 4, numbers of individuals possessing the indicated allele 1 are significantly higher among the ESNs under a dominant gene hypothesis at the *Card10, CDC42EP1,* and *GRAP2* loci, and the most highly significant difference between the ESN and HIV-infected individuals was observed at the *CDC42EP1* locus (P = 0.0043). In Table 4 The numbers of individuals possessing and not possessing allele 1 between the ESN and HIV-infected groups were compared by Fisher's exact probability test. 1 = homozygous for allele 1; ½ = heterozygous;, and 2 =, homozygous for allele 2 (alleles as listed in Table 3).

**Table 4**

| | | Genotype distribution among the HIV-exposed but uninfected individuals | | | | | Genotype distribution among the HIV-infected individuals | | | | | *P* |
|---|---|---|---|---|---|---|---|---|---|---|---|---|
| SNP ID | Linked locus | 1 | 1/2 | 2 | ND | Total | 1 | 1/2 | 2 | ND | Total | |
| 1088426 | *APOL3* | 17 | 34 | 22 | 1 | 74 | 16 | 40 | 21 | 0 | 77 | |
| 8713601 | *MYH9* | 40 | 26 | 6 | 2 | 74 | 51 | 21 | 4 | 1 | 77 | |
| 1841062 | *RABL4* | 2 | 16 | 55 | 1 | 74 | 1 | 24 | 52 | 0 | 77 | |
| 8956971 | *EA57_HUMAN* | 10 | 42 | 20 | 2 | 74 | 7 | 38 | 32 | 0 | 77 | |
| | *EA57_HUMAN* | | | | | | | | | | | |
| 25968036 | (exon1 coding) | 0 | 52 | 18 | 4 | 74 | 0 | 58 | 18 | 1 | 77 | |
| 2403433 | *IL2RB* | 49 | 19 | 5 | 1 | 74 | 52 | 23 | 2 | 0 | 77 | |
| 2403368 | *C1QTNF6* | 5 | 26 | 41 | 2 | 74 | 3 | 24 | 49 | 1 | 77 | |
| 15960075 | *RAC2* | 45 | 25 | 2 | 2 | 74 | 43 | 31 | 1 | 2 | 77 | |
| 2236051 | *RAC2* | 74 | 0 | 0 | 0 | 74 | 77 | 0 | 0 | 0 | 77 | |
| 25994985 | *CARD10* | 74 | 0 | 0 | 0 | 74 | 77 | 0 | 0 | 0 | 77 | |
| 25993567 | *CARD10* | 46 | 21 | 2 | 5 | 74 | 41 | 21 | 10 | 5 | 77 | 0.0313 |
| 19531 | *CARD10* | 44 | 11 | 0 | 5 | 60 | 41 | 13 | 2 | 4 | 60 | |
| 8957740 | *CARD10* | 42 | 15 | 0 | 3 | 60 | 45 | 10 | 0 | 5 | 60 | |
| 2491547 | *CDC42EP1* | 12 | 28 | 14 | 6 | 60 | 17 | 27 | 12 | 4 | 60 | |
| 2491542 | *CDC42EP1* | 34 | 34 | 4 | 2 | 74 | 30 | 30 | 17 | 0 | 77 | 0.0043 |
| 15875008 | *LGALS2* | 0 | 0 | 54 | 0 | 54 | 0 | 0 | 58 | 1 | 59 | |
| 2233479 | *POLR2F* | 13 | 35 | 25 | 1 | 74 | 16 | 36 | 24 | 1 | 77 | |
| 2501764 | *MAFF* | 13 | 30 | 29 | 2 | 74 | 20 | 31 | 24 | 2 | 77 | |
| 344103 | *GTPBP1 APOBEC3G* | 9 | 29 | 36 | 0 | 74 | 9 | 30 | 36 | 2 | 77 | |
| 2189646 | (exon4 coding) | 68 | 6 | 0 | 0 | 74 | 68 | 7 | 0 | 2 | 77 | |
| | *APOBEC3G* | | | | | | | | | | | |
| 25649193 | (exon6 conding) | 67 | 7 | 0 | 0 | 74 | 70 | 6 | 0 | 1 | 77 | |
| 2221682 | *RPL3* | 30 | 33 | 10 | 1 | 74 | 26 | 38 | 11 | 2 | 77 | |
| 2222537 | *GRAP2* promoter | 36 | 32 | 2 | 4 | 74 | 33 | 36 | 7 | 1 | 77 | |
| 2222563 | *GRAP2* (intron1) | 10 | 20 | 24 | 1 | 55 | 6 | 20 | 6 | 1 | 33 | 0. 0196 |
| 2467289 | *GRAP2* (intron2) | 3 | 12 | 58 | 1 | 74 | 2 | 19 | 54 | 2 | 77 | |
| 15530 | *GRAP2* (intron3) | 1 | 19 | 49 | 5 | 74 | 1 | 23 | 50 | 3 | 77 | |
| 2467292 | *GRAP2* (intron3) | 19 | 40 | 14 | 1 | 74 | 20 | 37 | 20 | 0 | 77 | |
| 11484908 | *GRAP2* (exon8 conding) | 74 | 0 | 0 | 0 | 74 | 76 | 0 | 0 | 1 | 77 | |
| 16318 | *GRAP2* (3' intron) | 52 | 20 | 2 | 0 | 74 | 48 | 26 | 1 | 2 | 77 | |
| 11882437 | *Q9UP9Q (TNRC6B)* | 28 | 36 | 8 | 2 | 74 | 30 | 33 | 13 | 1 | 77 | |
| 224082 | *NOVEL10 (LOC63929)* | 21 | 42 | 10 | 1 | 74 | 25 | 41 | 10 | 1 | 77 | |
| 2497323 | *TOB2* | 2 | 27 | 45 | 0 | 74 | 4 | 22 | 51 | 0 | 77 | |
| 2481122 | *NM 024821 (FLJ22349)* | 1 | 20 | 49 | 4 | 74 | 2 | 21 | 53 | 1 | 77 | |
| 2189968 | *BAFF-R* | 37 | 28 | 7 | 2 | 74 | 38 | 26 | 11 | 2 | 77 | |
| 2189972 | *C22orf18* | 6 | 28 | 37 | 3 | 74 | 8 | 28 | 40 | 1 | 77 | |
| 2468720 | *TCF20* | 3 | 27 | 43 | 1 | 74 | 4 | 29 | 43 | 1 | 77 | |
| 2986155 | *NM_170698 (dJ222E13. 2)* | 4 | 28 | 41 | 1 | 74 | 6 | 42 | 28 | 1 | 77 | 0.0218 |
| 1150511 | *A4GALT* | 26 | 20 | 4 | 0 | 50 | 26 | 26 | 6 | 0 | 58 | |

| | | | | | | | | | | | | |
|---|---|---|---|---|---|---|---|---|---|---|---|---|
| ND: not determined. | | | | | | | | | | | | |

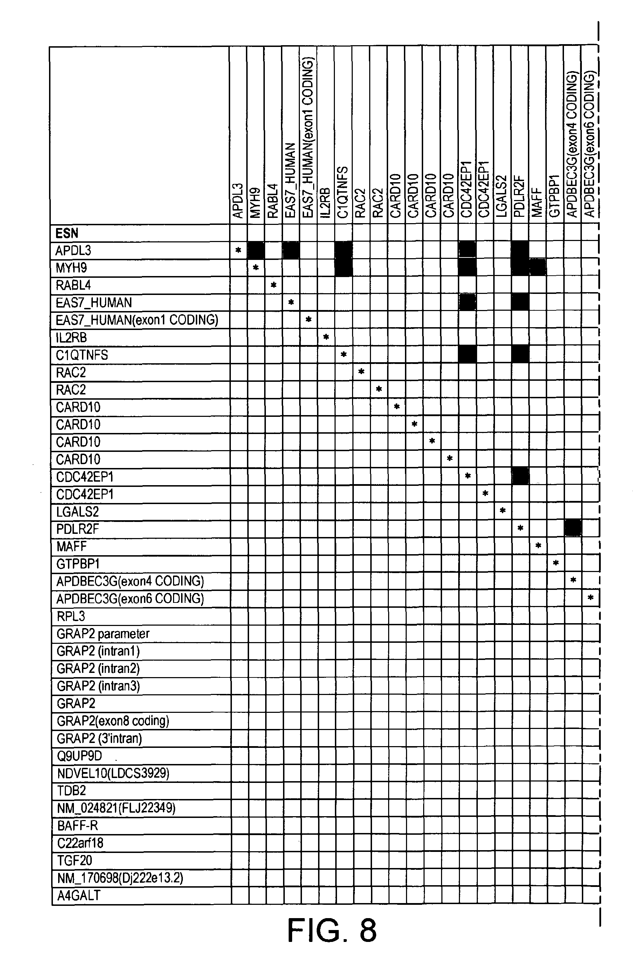

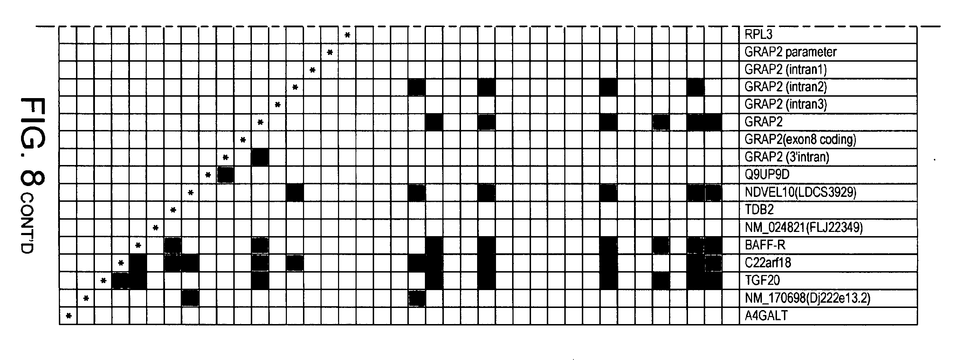

2001 [58] indicated that genotypes at the SNP loci throughout the chromosome 22 region between the *APOL3* and *A4GALT* are highly linked in the ESN individuals but not in the HIV-infected individuals. See Figures 8 and 9. The patterns of linkage disequilibrium are especially different between the ESN and HIV-infected individuals in the centromeric half of the region. It should be noted that there is an apparent disruption in linkage disequilibrium between the SNP loci linked to *Tob2* and *MN_024821* in the ESN group (Figure 8). This is consistent with the previously observed disruption in the linkage disequilibrium between the microsatellite markers at the D22S276 locus in the ESNs, because D22S276 is located between the above two genes (see Figure 3).

### Conclusions

Since the SNP loci at which the numbers of individuals possessing the indicated allele 1 are significantly different between the ESN and HIV-infected groups are physically close to the two genes, *Rac2* and *PSCD4,* whose expression levels after the HIV antigenic stimulation are higher in the ESN individuals (see Figure 10 in which the SNP loci linked to the *Card10* and *CDC42EP1* loci are shown with large brown arrows), and a strong linkage disequilibrium was observed between the SNP locus linked to *CDC42EP1* and the centromeric loci linked to *MYH9* and *C1QTNF6* encompassing *Rac2 and Card10* only among the ESNs, it is likely that a genetic polymorphism(s) closely linked to the *Card10* and *CDC42EP1* genotypes is associated with the observed high expression of the Rac2 and *PSCD4* genes.

### Example 4

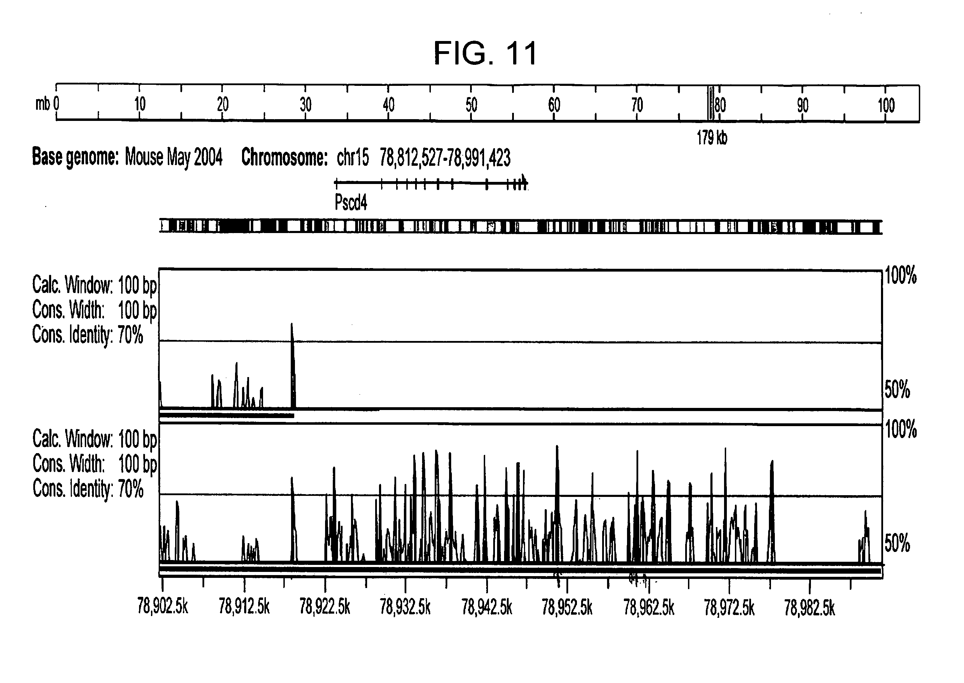

Gene expressions are controlled by promoter and enhancer sequences, and these controlling elements, unlike non-functional portions of a chromosome, are relatively conserved in their sequences between different species. In the case of our analyses, *Rac2* and *CSCD4* that are located next to each other on chromosome 22 but are in opposite translational directions (as shown in Fig 11) are both induced upon the HIV antigenic stimulation. Therefore, we postulated that a common enhancer sequence(s) may be involved in the induction of these genes. Thus, genomic sequences of the chromosomal region encompassing these two genes enlisted in the public genome database were compared between mice and humans using Genome Vista (http://genome.lbl.gov/vista/index.shtml).

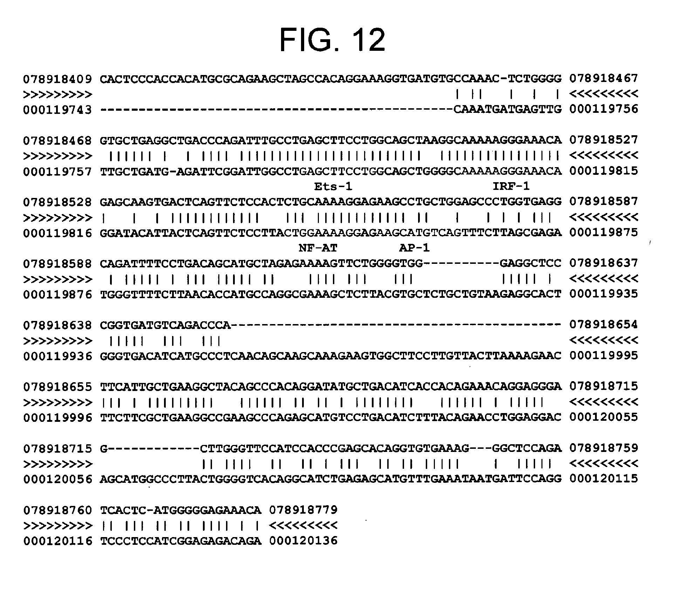

As shown in Figure 11, there are areas between *Rac2* and *PSCD4* where high sequence homologies of >50% were observed between the human and mouse genomes [64] Among them, the region nearer to the *PSCD4* gene (indicated with a red arrow in the above Figure) contains Ets-1, IRF-1, NFAT, and AP-1 binding-like motifs, indicating this region to be possible enhancer (Figure 12).

Thus, genomic sequences of these two regions were determined by using the PCR primers shown in Figure 13. The underlined sequences are PCR primers, and the oligonucleotides shown in red were used for sequencing. Yellow-coloured portions are areas showing greater than 70% of sequence identity over 100 base-pairs between humans and mice. We found two SNPs, one in the region 1 and the other in the regions 2, as highlighted in Figure 13 with blue.

Frequencies of individuals possessing the each allele were compared between the ESN and HIV-infected groups. At the region 2, 10 out of the 22 ESN individuals possessed the T to G SNP indicated above, while only 2 out of the 15 HIV-infected individual tested possessed the G allele at the same locus (*X*² = 4.2, *P* = 0.04), suggesting that these SNPs are likely to be associated with the observed higher expression of *Rac2* and *PSCD4* in ESNs.

### Conclusions

All the results shown here indicate that 1) genetic polymorphisms in the *Rac2-PSCD4-Card10-CDC42EP1* region of human chromosome 22 is associated with HIV-exposed but uninfected status, 2) *Rac2* and *PSCD4* that are functionally associated with T cell activation and at least the former has been associated with IFN-γ production from T helper cells, are expressed higher in the ESN than HIV-infected individuals after the stimulation of peripheral blood mononuclear cells with HIV-1 antigens, and 3) a T to G polymorphism in the putative enhancer region between the *Rac2* and *PSCD4* genes are apparently highly accumulated in the ESN individuals.

### References

1. O'Brien, S. J., Nelson, G. W., Winkler, C. A. & Smith, M. W. Polygenic and multifactorial disease gene association in man: Lessons from AIDS. Annu. Rev. Genet. 34, 563-591 (2000).
2. Dean, M. et al. Genetic restriction of HIV-1 infection and progression to AIDS by a deletion allele of the CCR5 structural gene. Science 273, 1856-1862 (1996).
3. Liu, R. et al. Homozygous defect in HIV-1 coreceptor accounts for resistance of some multiply-exposed individuals to HIV-1 infection. Cell 86, 367-377 (1996).
4. Samson, M. et al. Resistance to HIV-1 infection in Caucasian individuals bearing mutant alleles of the CCR-5 chemokine receptor gene. Nature 382, 722-725 (1996).
5. Martin, M. P. et al. Genetic acceleration of AIDS progression by a promoter variant of CCR5. Science 282, 1907-1911 (1998).
6. Smith, M. W. et al. Contrasting genetic influence of CCR2 and CCR5 receptor gene variants on HIV-1 infection and disease progression. Science 277, 959-965 (1997).
7. Winkler, C. et al. Genetic restriction of AIDS pathogenesis by an SDF-1 chemokine gene variant. Science 279, 389-393 (1998).
8. Carrington, M. et al. HLA and HIV: Heterozygote advantage and B*35-Cw*04 disadvantage. Science 283, 1748-1752 (1999).
9. Shin, H. D. et al. Genetic restriction of HIV-1 infection and AIDS progression by promoter alleles of interleukin 10. Proc. Natl. Acad. Sci. USA. 97,14467-14472 (2000).
10. Beyrer, C. et al. Epidemiologic and biologic characterization of a cohort of human immunodeficiency virus type 1 highly exposed, persistently seronegative female sex workers in northern Thailand. J. Infect. Dis. 79, 59-68 (1999).
11. Martin, M. P. et al. Epistatic interaction between KIR3DS1 and HLA-B delays the progression to AIDS. Nature Genet. 31, 429-434 (2002).
12. Rowland-Jones, S. L. & McMichael, A. Immune responses in HIV-exposed seronegatives: Have they repelled the virus? Curr. Opin. Immunol. 7, 448-455 (1995).
13. Shearer, G. M. & Clerici, M. Protective immunity against HIV infection: Has nature done the experiment for us? Immunol. Today 17, 21-24 (1996).
14. Mazzoli, S. et al. HIV-specific mucosal and cellular immunity in HIV-seronegative partners of HIV-seropositive individuals. Nature Med. 3, 1250-1257, 1997.
15. Kaul, R. et al. HIV-1 specific mucosal IgA in a cohort of HIV-1-resistent Kenyan sex workers. AIDS 13, 23-29 (1999).
16. Biasin, M. et al. Mucosal and systemic immune activation is present in human immunodeficiency virus-exposed seronegative women. J. Infect. Dis. 182, 1365-1374 (2000).
17. Belec, L. et al. Cervicovaginal secretory antibodies to HIV type 1 that block viral transcytosis through epithelial barriers in highly exposed HIV-1-seronegative African women. J. Infect. Dis. 184, 1412-1422 (2001).
18. Mazzoli, S. et al. Human immunodeficiency virus (HIV)-specific IgA and HIV neutralizing activity in the serum of exposed seronegative partners of HIV-seropositive persons. J. Infec. Dis. 180, 871-875 (1999).
19. Locaputo, S. et al. Mucosal and systemic HIV-specific immunity in HIV-exposed but uninfected heterosexual males. AIDS 17, 531-538 (2002).
20. Letvin, N. et al. Potent, protective anti-HIV immune responses generated by bimodal HIV envelope DNA plus protein vaccination. Proc. Natl. Acad. Sci. USA 94, 9378-9383 (1997).
21. Mascola, J.R. et al. Protection of macaques against pathogenic simian/human immunodeficiency virus 89.6PD by passive transfer of neutralizing antibodies. J. Virol. 73, 4009-4018 (1998).
22. Mascola, J.R. et al. Protection of macaques against vaginal transmission of pathogenic HIV-1/SIV chimeric virus by passive infusion of neutralizing antibodies. Nat. Med. 6, 207-210 (2000).
23. Earl, P.L. et al. Immunogenicity and protective efficacy of oligomeric human immunodeficiency virus type 1 gp140. J. Virol. 75, 645-653 (2001).
24. Harris, R.S., Bishop, K.N., Sheehy, A.M., Craig, H.M., Peterson-Mahrt, S.K., Watt, I.N., Neuberger, M.S. Malim, M.H. DNA deamination mediates innate immunity to retroviral infection. Cell 113, 803-809 (2003).
25. Zhang, H., Yang, B., Pomerantz, R.J., Zhang, C., Arunachalam, S.C., Gao, L. The cytidine deaminase CEM15 induces hypermutation in newly synthesized HIV-1 DNA. Nature 424, 94-98 (2003).
26. Harris, R. S., Sheehy, A. M., Craig, H. M., Malim, M. H., & Neuberger, M. S. DNA reamination: not just a trigger for antibody diversification but also a mechanism for defense against retroviruses. Nat. Immunol. 4, 641-643 (2003)
27. Bishop, K.N., Holmes, R.K., Sheehy, A.M., Davidson, N.O., Cho, S.J., Malim, M.H. Cytidine deamination of retroviral DNA by diverse APOBEC proteins. Curr. Biol. 14, 1392-1396 (2004)
28. Mangeat, B., Turelli, P., Caron, G., Friedli, M., Perrin, L., Trono, D. Broad antiretroviral defence by human APOBEC3G through lethal editing of nascent reverse transcripts. Nature 424, 99-103 (2003)
29. Sheehy, A.M., Gaddis, N.C., Choi, J.D., Malim, M.H. Isolation of a human gene that inhibits HIV-1 infection and is suppressed by the viral Vif protein. Nature 418, 646-650 (2002).
30. Marin, M., Rose, K.M., Kozak, S.L., Kabat, D. HIV-1 Vif protein binds the editing enzyme APOBEC3G and induces its degradation. Nat. Med. 9, 1398-1403 (2003)
31. Yu, X., Yu, Y., Liu, B., Luo, K., Kong, W., Mao, P., Yu, X.F. Induction of APOBEC3G ubiquitination and degradation by an HIV-1 Vif-Cul-SCF complex. Science 302, 1056-1060 (2003).
32. Conticello, S.G., Harris, R.S., Neuberger, M.S. The Vif protein of HIV triggers degradation of human antiretroviral DNA deaminase APOBEC3G. Curr. Biol. 13, 1009-2013 (2003).
33. Stopak, K., de Noronha, C., Yonemoto,W., Greene, W.C., HIV-1 Vif blocks the antiretroviral activity of APOBEC3G by impairing both its translation and intracellular stability. Mol. Cell 12, 591-601 (2003)
34. Gabzda, D. H., Lawewncw, K., Langhoff, E., Terwillinger, E., Dorfman, T., Haseltine, W. A., & Sodroski, J. Role of vif in replication of human immunodeficiency virus type 1 in CD4+ T lymphocytes. J. Virol. 66, 6489-6495 (1992)
35. Zheng, Y.H., Irwin, D., Kuroosu, T., Tokunaga, K., Sata, T., Peterlin, B.M. Human APOBEC3F is another host factor that blocks human immunodeficiency virus type 1 replication. J. Virol. 78, 6073-6076 (2004).
36. Liddament, M.T., Brown, W.L., Schumacher, A.J., Harris, R.S. APOBEC3F properties and hypermutation preferences indicate activity against HIV-1 in vivo. Curr Biol. 14, 1385-1391 (2004)
37. Wiegand, H.L., Doehle, B.P., Bogerd, H.P., Cullen, B.R. A second human antiretroviral factor, APOBEC3F, is suppressed by the HIV-1 and HIV-2 Vif proteins. EMBO 23, 2451-2458 (2004)
38. Teich, N., Wyke, J., Mak, T., Bernstein, A. & Hardy, W. Pathogenesis of retrocirus-induced disease. RNA Tumor Viruses, 2nd ed. Cold Spring Harbor Laboratory, New York (19872), pp785-998.
39. Kabat, D. Molecular biology of Friend viral erythroleukemia. Curr. Top. Microbiol. Immunol. 148, 1-42 (1989).
40. Chesebro, B., Miyazawa, M. & Britt, W.J. Host genetic control of spontaneous and induced immunity to Friend murine retrovirus infection. Annu. Rev. Immunol. 8, 477-499 (1990).
41. Hoatlin, M.E. & Kabat, D. Host-range control of a retroviral disease: Friend erythroleukemia. Trends Microbiol. 3, 51-57 (1995).
42. Best, S., Le Tissier, P. Towers, G. & Stoye, J.P. Positional cloning of the mouse retrovirus restriction gene Fv1. Nature 382, 826-829 (1996).
43. Persons, D.A. et al. Fv2 encodes a truncated form of the Stk receptor tyrosine kinase. Nat. Genet. 23, 159-165 (1999).
44. Ikeda, H., Laigret, F., Martin, M.A. & Repaske, R. Characterization of a molecularly cloned retroviral sequence associated with Fv-4 resistance. J. Virol. 55, 768-777 (1985).
45. Ikeda, H. & Sugimura, H. Fv-4 resistance gene: a truncated endogenous murine leukaemia virus with ecotropic interference properties. J. Virol. 63, 5405-5412 (1989).
46. Miyazawa, M., Nishio, J. & Chesebro, B. Genetic control of T cell responsiveness to the Friend murine leukaemia virus envelope antigen. Identification of the class II loci of H-2 as immune response genes. J. Exp. Med. 168, 1587-1605 (1988).
47. lwashiro, M. et al. Multiplicity of virus-encoded helper T-cell epitopes expressed on FBL-3 tumor cells. J. Virol. 67, 4533-4542 (1993).
48. Peterson, K.E., lwashiro, M., Hasenkrug, K.J. & Chesebro, B. Major histocompatibility complex class I gene controls the generation of gamma interferon-producing CD4+ and CD8+ T cells important for recovery from Friend retrovirus-induced leukaemia. J. Virol. 74, 5363-5367 (2000).
49. Miyazawa, M., Nishio, J., Wehrly, K., David, C.S. & Chesebro, B. Spontaneous recovery from Friend retrovirus-induced leukaemia. Mapping of the Rfv-2 gene in the Q/TL region of mouse MHC. J. Immuno. 148, 1964-1976 (1992).
50. lwanamil, N., Niwa, A., Yasutomi, Y., Tabata, N. & Miyazawa, M. Role of natual killer cells in resistance against Friend retrovirus-induced leukaemia. J. Virol. 75, 3152-3163 (2001).
51. Chesebro, B. & Wehrly, K. Studies on the role of the host immune response in recovery from Friend virus leukaemia. I. Antiviral and antileukemia cell antibodies. J. Exp. Med. 143, 73-84 (1976).
52. Hasenkrug, K.J. et al. Chromosome mapping of Rfv3, a host resistance gene to Friend murine retrovirus. J. Virol. 69, 2617-2620 (1995).
53. Super, H.J. et al. Fine mapping of the Friend retrovirus resistance gene, Rfv3, on mouse chromosome 15. J. Virol. 73, 7848-7852 (1999).
54. Doig, D. & Chesebro, B. Anti-Friend virus antibody is associated with recovery from viremia and loss of viral leukaemia cell-surface antigens in leukemic mice. Identification of Rfv-3 as a gene locus influencing antibody production. J. Exp. Med. 150, 10-19 (1979).
55. Miyazawa, M., Nishio, J., Wehrly, K. & Chesebro, B. Influence of MHC genes on spontaneous recovery from Friend retrovirus-induced leukaemia. J. Immunol. 148, 644-646 (1992).
56. (Horrevoets, A. J., et al. Vascular endothelial genes that are responsive to tumor necrosis factor-alpha in vitro are expressed in atherosclerotic lesions, including inhibitor of apoptosis protein-1, stannin, and two novel genes. Blood 1999, 93:3418-31.)
57. (Gu, Y., et al. Hematopoietic cell regulation by Rac1 and Rac2 guanosine triphosphatases. Science 2003 302:445-9.),
58. (Wang, L., et al. Card10 is a novel caspase recruitment domain/membrane-associated guanylate kinase family member that interacts with BCL10 and activates NF-kappa B. J. Biol. Chem. 2001, 276:21405-9.)
59. (Ikematsu, N., et al. Tob2, a novel anti-proliferative Tob/BTG1 family member, associates with a component of the CCR4 transcriptional regulatory complex capable of binding cyclin-dependent kinases. Oncogene 1999, 18:7432-41)
60. (Hentges, K. E, et al. Tnfrsf13c (Baffr) is mis-expressed in tumors with murine leukemia virus insertions at Lvis22. Genomics 2002, 80:204-212.).
61. YasuyoshiKanari,MarioClerici,HiroyukiAbe,HiroyukiKawabata,DariaTra battoni,SergioLoCaputo,FrancescoMazzotta,HironoriFujisawa,AtsukoNi wa,Chiakilshihara,YumikoA.Takeiand Masaaki Miyazawa Genotypes at chromosome22q12-13 areassociatedwithHIV-1 exposed but uninfected status in Italians AIDS 2005, 19: 1015-1024
62. (Li, B., Yu, H., Zheng, W., Voll, R., Na, S., Roberts, A. W., Williams, D. A., Davis, R. J., Ghosh, S., and Flavell, R. A. Role of the guanosine triphosphatase Rac2 in T helper 1 cell differentiation. Science 288: 2219-2222, 2000).
63. (Dixon, B., Mansour, M., Pohajdak, B. Assignment of human B2-1 gene (D17S811 E) to chromosome 17qter by PCR analysis of somatic cell hybrids and fluorescence in situ hybridization. Cytogenet. Cell Genet. 63: 42-44, 1993).
64. (Hardison, Trends Genet. 16: 369, 2000), 65 (Loots et al., Science 288: 136, 2000)].
65. (Gu, Y., et al. Hematopoietic cell regulation by Rac1 and Rac2 guanosine triphosphatases. Science 2003 302:445-9.),
66. =(Hentges, K. E, et al. Tnfrsf13c (Baffr) is mis-expressed in tumors with murine leukemia virus insertions at Lvis22. Genomics 2002, 80:204-212.).
67. YasuyoshiKanari,MarioClerici,HiroyukiAbe,HiroyukiKawabata,DariaTra battoni,SergioLoCaputo,FrancescoMazzotta,HironoriFujisawa,AtsukoNi wa,Chiakilshihara,YumikoA.Takeiand Masaaki Miyazawa Genotypes at chromosome22q12-13 areassociatedwithHIV-1 exposed but uninfected status in Italians AIDS 2005, 19: 1015-1024
68. (Dixon, B., Mansour, M., Pohajdak, B. Assignment of human B2-1 gene (D175811E) to chromosome 17qter by PCR analysis of somatic cell hybrids and fluorescence in situ hybridization. Cytogenet. Cell Genet. 63: 42-44, 1993).
69. (Hardison, Trends Genet. 16: 369, 2000), 65 (Loots et al., Science 288: 136, 2000)].

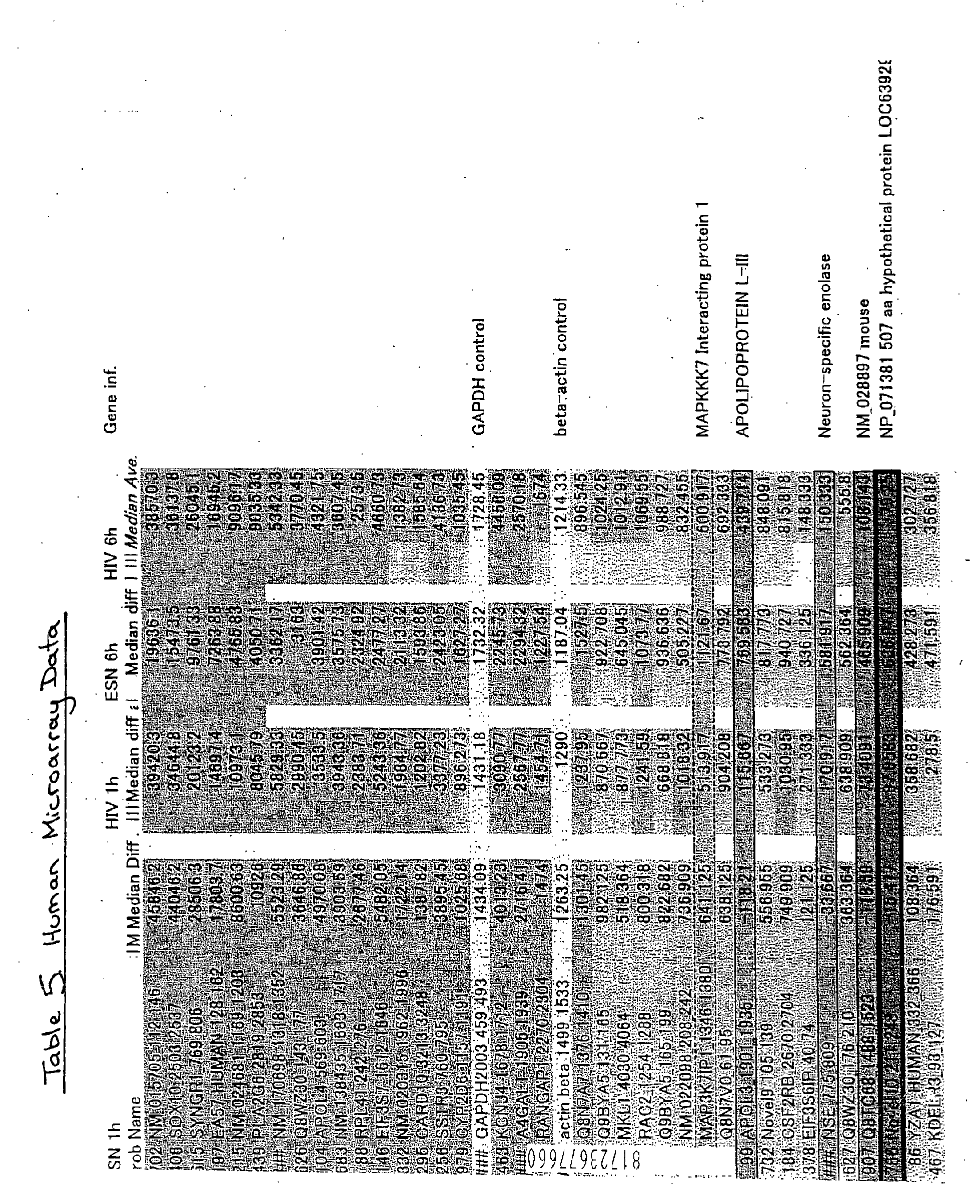

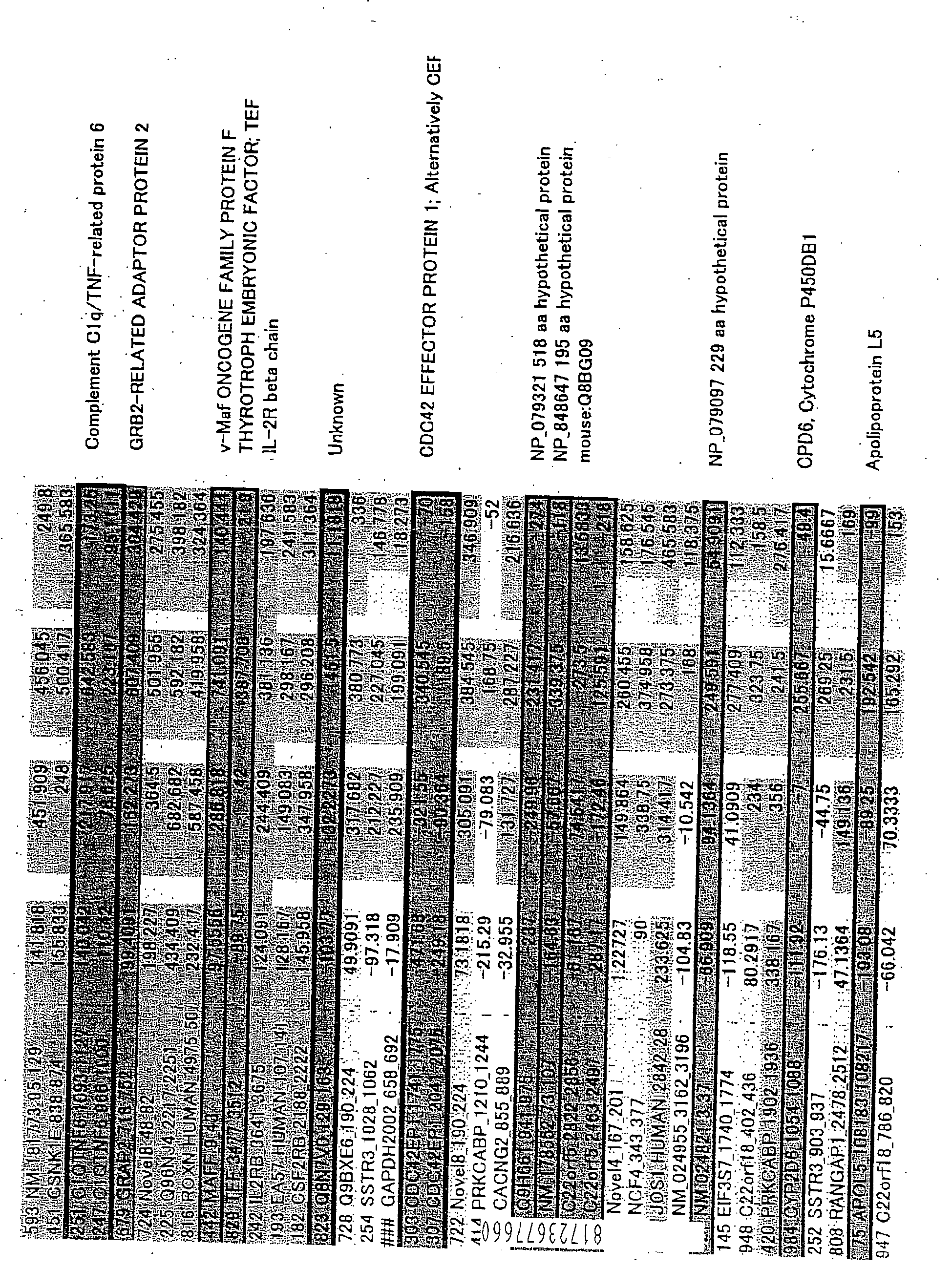

## Claims

1. An isolated nucleic acid sequence encoding a gene located in the region of human chromosome 22 between the loci D22S277 and D22S423 or a functional fragment thereof.

2. An isolated nucleic acid sequence encoding a gene located in the region of human chromosome 22 that is syntenic to the region of mouse chromosome 15 between the loci D15Mit68 and D15Mit107 or a functional fragment thereof.

3. An isolated nucleic acid encoding a gene which is a homologue or orthologue of a mouse gene selected from the list consisting of *Q8CCA5 (APOL3), 2600013G09Rik (RABL4), Rac2, Card10, D230019K20Rik (KA93_Human), PSCD4, CDC42EP1, Q9D6D6 (Tob2), 2610019103Rik (C22orf18)* and *Tnfrsf13c (Baffr),.*

4. An isolated nucleic acid sequence encoding a gene selected from the group consisting of *Apol3, MYH9, IL2RB, C1QTNF6, RAC2, CDC42EP1, LGALS2, POLR2F, MAFF, GTPBP1, RPL3, GRAP2,* Q9UP9Q, RABL4, *KA93_HUMAN C22orf18* human *Baffr,* BAFF-R, C22orf18, CSNK1E, *CDC42EP1, APOBEC3G, TNFRSF13C, EA57_HUMAN, CARD10, PSCD4, NM_152669, NM_024821 (FLJ22349), NM_170698 (Dj222e13.2), Novel 9, NOVEL 10 (LOC63929), TOB2, TCF20, A4GALT and RBX1* or a functional fragment thereof.

5. An isolated nucleic acid sequence encoding gene selected from the region *Rac2-PSCD4-Card10-CDC42EP1* or a functional fragment thereof.

6. An isolated nucleic acid sequence encoding either gene *Rac2 and PSCD4* or a functional fragment thereof

7. The nucleic acid sequence according to SEQ ID No: 1 or a functional fragment thereof.

8. The nucleic acid sequence according to SEQ ID No: 2 or a functional fragment thereof.

9. A pharmaceutical preparation comprising a nucleic acid according to any one of Claims 1 to 8 and a pharmaceutically acceptable carrier or vehicle.

10. Use of a nucleic acid according to any one of Claims 1 to 8 in medicine.

11. Use of a nucleic acid according to any one of Claims 1 to 8 in a vaccine.

12. Use of a nucleic acid according to any one of Claims 1 to 8 in the preparation of a medicament for the treatment or prophylaxis of infection.

13. Use of a nucleic acid according to any one of Claims 1 to 8 in the preparation of a medicament for the treatment or prophylaxis of viral infection.

14. Use according to Claim 11, in which the virus is a retrovirus.

15. Use according to Claim 12 or Claim 13, in which the virus is HIV.

16. A method of treating or preventing infection, the method comprising augmenting or inhibiting expression of one or more genes encoded by a nucleic acid according to any one of Claims 1 to 8.

17. Use of a nucleic acid according to any one of Claims 1 to 8 in gene therapy.

18. Use of a nucleic acid according to any one of Claims 1 to 8 in the preparation of a medicament for use in gene therapy.

19. Use of a nucleic acid according to any one of Claims 1 to 8 in a method of determining a pre-disposition or resistance to infection.

20. Use of a nucleic acid according to any one of Claims 1 to 8 in an in vitro method of screening compounds for a compound which has an effect on expression of said nucleic acid.

21. A polypeptide encoded by a nucleic acid according to any one of Claims 1 to 8, or a functional fragment, homologue or tertiary product thereof.

22. A polypeptide encoded by SEQ Id No: 1 or by SEQ ID No: 2.

23. A polypeptide according to Claim 21 or Claim 22, in which the polypeptide is produced synthetically.

24. Use of one or more polypeptides according to any one of Claims 21 to Claim 22 in medicine.

25. Use of one or more polypeptides according to any one of Claims 21 to Claim 22 in the manufacture of a medicament for the treatment or prevention of infection.

26. Use according to Claim 21 and 22, wherein the infection is a viral infection.

27. Use according to Claims 24 and 25 wherein the virus is a retrovirus.

28. Use according to Claim 24, 25 and 26, wherein the virus is HIV.

29. A pharmaceutical composition comprising one or more polypeptides according to any one of Claims 21 to Claim 23 and a pharmaceutically acceptable carrier or vehicle.

30. A pharmaceutical composition according to Claim 29 which is a vaccine.

31. A method of treating or preventing infection, the method comprising administering a pharmaceutically effective amount of one or more polypeptides according to any one of Claims 21 to Claim 22 to an individual in need of such treatment.

32. A method of treating or preventing infection, the method comprising augmenting or inhibiting expression of one or more genes encoding a polypeptide according to any one of Claims 21 to Claim 22 in an individual in need of such treatment.

33. Use of a polypeptide according to any one of Claims 21 to Claim 22 in a method of screening of compounds for functional homologues of said polypeptides.

34. Use of a polypeptide according to any one of Claims 21 to Claim 22 in a method of testing for susceptibility to infection

35. An antibody to a polypeptide according to any one of Claims 21 to Claim 23.

36. Use of an antibody according to Claim 35 in a method of screening compounds for functional homologues of said polypeptides.

37. An isolated nucleic acid probe as shown in Figure 14.

38. Use of any one of the probes of Claim 34 in an assay for the determination of a resistance to infection.

39. Use of any one of the probes of Claim 34 in the determination of a compound for modifying the expression of a gene according to any one of Claims 1 to 8

40. Use of any one of the probes of Claim 37 in the determination of a compound for modifying the expression of a polypeptide according to any one of Claims 21-22

41. Use of any one of the probes of Claim 37 in a method of treating or preventing infection.

42. Use of any one of the probes according to Claim 37 in the preparation of a medicament for the treatment and prophylaxis of viral infection.

43. Use according to Claim 42 in which the viral infection is HIV infection.

44. Use of any one of the probes according to Claim 42 in the treatment or prevention of AIDS.

45. Augmentation of expression of one or more genes encoding cytokines for the treatment or prevention of infection.

46. Use according to Claim 45 in which the infection is HIV infection.

47. Use of an isolated nucleic acid sequence according to any one of Claims 1 to 8 in a method of determining disease progression in an infected patient.

48. Use of an isolated nucleic acid sequence according to any one of Claims 1 to 8 in the modification or inhibition of viral infection in infected patient.

49. Use of polypeptide or fragments thereof according to any one of Claims 21-22 in the modification or inhibition of viral infection and clinical disease in infected patient.


SEQUENCE LISTING

<110> ImmunoClin Ltd
      Osaka Industrial Promotion Organization
      Toppan Printing Company
      Miyazawa, Masaaki
      Clerici, Mario
      Irie, Shinji

<120> Resistance Genes

<130> JMH/9249EP/D00

<140> EP10185349.7
<141> 2005-12-28

<150> GB 0428301.6
<151> 2004-12-24

<160> 27

<170> PatentIn version 3.1

<210> 1
<211> 721
<212> DNA
<213> Homo sapiens

<400> 1
tgcacccagc caactactga agttctatat cagatcttta tacccagcag agcaaatcat      60
ccactcttga atgttttcaa aattgttatc gttaccactg acactttgct ctttcatata     120
aagttcataa taagcttgtc aagttccaag aaaaactgtg tgaggattct gattagaatt     180
gtattcaatt tctagaggaa cttggggaga agtgacatct ttatatattg agtcttcctg     240
tccattaaca tggtatattg cttctttata tctttcaatt aagttttata atttcccccc     300
ggaaaggtca tgcacatttt ttgaaagatt cattccttga taccttatta ctttcagtgc     360

cattataact aatatttttt aaattgaatt ttctatttgt tgcccatacg tagggaaaat 420

gtcacatttt gtatattgaa cttatataga aacctggatg gaaattctca ttctaattta 480

tgtaaagatt ttgctcctaa caagtctaca gctgtcttta tagacagtct tatcatttat 540

gaaaagtgac agttttattt cttccttccc aatttttata acgttaattc cttggcttgt 600

ctagatcttt tattacaggg ctgaagggaa tcagtgctgg caggcaacct tgtcttattt 660

ctgattttaa agagactgtt tctaatgttt caccattgaa cctgatgtct actgcaggct 720

t 721

<210> 2

<211> 773

<212> DNA

<213> Homo sapiens

<400> 2
ttttaagtgt ggtgtcccat gatggatatc tttattctgc ctttcttcct tccctcctcc 60

tttcctttta tattgagaat ctccatctcc cacatacaca aaaagctagg aatacaaaag 120

tagaagatca agaccaatga gaaaagcaga gggaaatatg ccaaatgatg agttgttgct 180

gatgagattc ggattggcct gagcttcctg gcagctgggg caaaaaggga aacaggatac 240

attactcagt tctccttact ggaaaaggag aagcatgtca gtttcttagc gagatgggtt 300

ttcttaacac catgccaggc gaaagctctt acgtgctctg ctgtaagagg cactgggtga 360

catcatgccc tcaacagcaa gcaaagaagt ggcttccttg ttacttaaaa gaacttcttc 420

gctgaaggcc gaagcccaga gcatgtcctg acatctttac agaacctgga ggacagcatg 480

gcccttactg gggtcacagg catctgagag catgtttgaa ataatgattc caggtccctc 540

catcggagag acagactcag aacttgggag tctatatctt aacagatacc ccaggtgagg 600

ctggtgtgta tccaggtttg agtttccctc cctaagatca tctttcctaa atctcaagaa 660

aacacagcca gcccctgtct tctgcctttg ctccctctga tcttcccaag ctgctgagac 720

attccacggt atccgcattt tacagatgga aaggctgagc ctgactgatg ctg 773

<210> 3

<211> 35

<212> DNA

<213> Artificial sequence

<220>

<223> Synthetic sequence: Primer

<400> 3
ctctcaccca gagaggcctg aaaaccgtgt tcgac 35

<210> 4

<211> 35

<212> DNA

<213> Artificial sequence

<220>

<223> Synthetic sequence: Primer

<400> 4
ccctgccatt tcttaagact ttctgctcca ctcac 35

<210> 5

<211> 35

<212> DNA

<213> Artificial sequence

<220>

<223> Synthetic sequence: Primer

<400> 5
ctccctgtgg cgtttcttag cagatggctg cagag 35

<210> 6

<211> 35

<212> DNA

<213> Artificial sequence

<220>

<223> Synthetic sequence: Primer

<400> 6
tgagtgtctc agaagtgtgc tcctctggcc tcagt 35

<210> 7

<211> 35

<212> DNA

<213> Artificial sequence

<220>

<223>  Synthetic sequence: Primer

<400>  7
attttctacg cctctgggga tatctgctca gccaa                                    35

<210>  8

<211>  35

<212>  DNA

<213>  Artificial sequence

<220>

<223>  Synthetic sequence: Primer

<400>  8
caggccagat ggttgctgcc acaacttgtg tacct                                   35

<210>  9

<211>  35

<212>  DNA

<213>  Artificial sequence

<220>

<223>  Synthetic sequence: Primer

<400>  9
atcctgagct gcacttacct gtgagagtct tcaaa                                   35

<210>  10

<211>  35

<212>  DNA

<213>  Artificial sequence

<220>

<223>  Synthetic sequence: Primer

<400>  10
atcgatggtc ctgcttgcca gagagttcct ctacc                                   35

<210>  11

<211>  35

<212>  DNA

<213>  Artificial sequence

<220>
<223>  Synthetic sequence: Primer

<400>  11
agattgactc ggtgaaatac ctggagtgct cagct                                35

<210>  12
<211>  35
<212>  DNA
<213>  Artificial sequence

<220>
<223>  Synthetic sequence: Primer

<400>  12
aactgccgca tgtggaagca tctacccgtt cactc                                35

<210>  13
<211>  35
<212>  DNA
<213>  Artificial sequence

<220>
<223>  Synthetic sequence: Primer

<400>  13
ttgcacacct tccacagggt caggatggag tagct                                35

<210>  14
<211>  35
<212>  DNA
<213>  Artificial sequence

<220>
<223>  Synthetic sequence: Primer

<400>  14
caggaggaga ccttgtggga catctgagga catcc                                35

<210>  15

<211>  35

<212>  DNA

<213>  Artificial sequence

<220>

<223>  Synthetic sequence: Primer

<400>  15
ctgcctctgt cacctcattg gtccatgaag gagca                                35

<210>  16

<211>  35

<212>  DNA

<213>  Artificial sequence

<220>

<223>  Synthetic sequence: Primer

<400>  16
ggcataagtg gctgttctgg gagtcattcc aaaga                                35

<210>  17

<211>  35

<212>  DNA

<213>  Artificial sequence

<220>

<223>  Synthetic sequence: Primer

<400>  17
cccagcatct cgagaccctc tgcagaagta ttatc                                35

<210>  18

<211>  35

<212>  DNA

<213>  Artificial sequence

<220>

<223>  Synthetic sequence: Primer

<400>  18
tcatgcttgg agagatagag tcttggccat ggctt                                35

<210>  19

<211>  35

<212>  DNA

<213>  Artificial sequence



<220>

<223>  Synthetic sequence: Primer

<400>  19
tctgaggaca tccgcagatt cttgtcagcc tgtga                                35

<210>  20

<211>  35

<212>  DNA

<213>  Artificial sequence



<220>

<223>  Synthetic sequence: Primer

<400>  20
tctctggagg tctgcaagca ctggtagtga tattg                                35

<210>  21

<211>  35

<212>  DNA

<213>  Artificial sequence



<220>

<223>  Synthetic sequence: Primer

<400>  21
agaagggaga gtttcgtgca ctgtggttaa cagga                                35

<210>  22

<211>  35

<212>  DNA

<213>  Artificial sequence



<220>

<223>  Synthetic sequence: Primer

<400>  22
aagaccactg acgagcagtc ccatggtgat aaagg                                35

<210>  23

<211>  371

<212>  DNA

<213>  Mus musculus

<400>  23
cactcccacc acatgcgcag aagctagcca caggaaaggt gatgtgccaa actctggggg      60

tgctgaggct gacccagatt tgcctgagct tcctggcagc taaggcaaaa agggaaacag     120

agcaagtgac tcagttctcc actctgcaaa aggagaagcc tgctggagcc ctggtgaggc     180

agattttcct gacagcatgc tagagaaaag ttctggggtg ggaggctccc ggtgatgtca     240

gacccattca ttgctgaagg ctacagccca caggatatgc tgacatcacc acagaaacag     300

gagggagctt gggttccatc cacccgagca caggtgtgaa agggctccag atcactcatg     360

ggggagaaac a                                                          371

<210>  24

<211>  394

<212>  DNA

<213>  Homo sapiens

<400>  24
caaatgatga gttgttgctg atgagattcg gattggcctg agcttcctgg cagctggggc      60

aaaaagggaa acaggataca ttactcagtt ctccttactg gaaaaggaga agcatgtcag     120

tttcttagcg agatgggttt tcttaacacc atgccaggcg aaagctctta cgtgctctgc     180

tgtaagaggc actgggtgac atcatgccct caacagcaag caaagaagtg gcttccttgt     240

tacttaaaag aacttcttcg ctgaaggccg aagcccagag catgtcctga catctttaca     300

gaacctggag gacagcatgg cccttactgg ggtcacaggc atctgagagc atgtttgaaa     360

taatgattcc aggtccctcc atcggagaga caga                                 394

<210>  25

<211>  63

<212>  DNA

<213>  Artificial sequence

<220>

<223>  Synthetic sequence: T7-oligo (dT) 24 primer

<400>  25
gcccagtgaa ttgtaatacg actcactata gggaggcggt tttttttttt tttttttttt      60

ttt                                                                    63

<210>  26

<211>  39

<212>  DNA

<213>  Artificial sequence

<220>

<223>  Synthetic sequence: Control probe

<400>  26
aggtctgtgt gatcatcttt gaccatatag attgcctct                             39

<210>  27

<211>  37

<212>  DNA

<213>  Artificial sequence

<220>

<223>  Synthetic sequence: Control probe

<400>  27
gaacccacta agatcaaata gggtgatgct ggttctg                               37